# EUROPEAN PATENT SPECIFICATION

(11) **EP 0 775 688 B1**
(45) Date of publication and mention of the grant of the patent: **05.04.2000**
(21) Application number: 96118532.9
(22) Date of filing: 19.11.1996
(51) Int. Cl.: C07C 209/60, C07B 43/04, C07C 227/06, C07C 303/30, C07C 221/00, C07C 231/12

(54) **Method for producing addition reaction product of amines and alpha, beta-unsaturated compounds**
Verfahren zur Herstellung von Additionsprodukten aus Aminen und Alpha-Beta ungesättigten Verbindungen
Procédé pour la préparation de produits d'une réaction d'addition entre des amines et des composés alpha-bêta-insaturés

(30) Priority: 21.11.1995 JP 30253295
(43) Date of publication of application: 28.05.1997
(73) Proprietor: Fuji Photo Film Co., Ltd., Kanagawa-ken (JP)
(72) Inventor: Inaba, Tadashi, Minami-Ashigara-shi, Kanagawa-ken (JP); Okada, Hisashi, Minami-Ashigara-shi, Kanagawa-ken (JP); Suzuki, Ryo, Minami-Ashigara-shi, Kanagawa-ken (JP)
(74) Representative: Hansen, Bernd, Dr. Dipl.-Chem.

(56) References cited:
- EP-A- 0 376 462
- TETRAHEDRON LETTERS, vol. 36, no. 2, 1995, OXFORD GB, pages 233-236, XP000645043 JENNER, GERARD: "Catalytic high pressure synthesis of hindered Beta-amino ester"
- "HOUBEN-WEYL, METHODEN DER ORGANISCHEN CHEMIE, vol. XI/1, pages 267 - 293 " 1957 , GEORG THIEME VERLAG , STUTTGART, DE XP002026197 * page 267, paragraph 2 *
- CHEMISTRY LETTERS, 1994, pages 827-830, XP000644690 MATSUBARA SEIJIRO ET AL.: "Lanthanoid triflate Catalyzed conjugate additions of amines to alpha,beta-unsaturated esters."
- JOURNAL OF THE AMERICAN CHEMICAL SOCIETY, vol. 72, no. 11, 1950, DC US, pages 5357-5361, XP002025852 ARTHUR E. MARTELL ET AL.: "The preparation and properties of some N,N'-Disubstituted-ethylenediaminedipropio nic acids"

## Description

The present invention relates to a method for producing an amine compound by introducing a cyanoethyl group, carboxyethyl group, sulfonylethyl group, acylethyl group, carbamoylethyl group or nitroethyl group into a compound having an amino group. The amine compound produced by the method of the present invention is usable in the fields of, for example, medical preparations, cosmetic preparations, soaps, detergents, cleaning compositions, analysis of materials, coating of metallic materials, platings, catalysts, colloid chemistry, photographs and liquid crystals. In addition, these amine compounds are useful also as intermediates of them.

It is known that in adding an amine to an α , β -unsaturated compound by the addition reaction, the reaction proceeds relatively rapidly only when the α , β -unsaturated compound has no substituent in the α -position and/or β -position [see, for example, Journal of the American Chemical Society, vol. 72, 5357 to 5361 (1950)]. However, the reactivity is extremely low and a long time is necessitated for the addition reaction when the α , β -unsaturated compound to be reacted has a substituent.

BF₃ · OEt₂, TiCl₄, SnCl₄, neutral Al₂O₃ and Triton B well-known as catalysts for Michael's reaction did not accelerate the addition of reaction.

Tetrahedron Letters, Vol 36, No. 2, pp 233-236, 1995, relates to the catalytic high pressure synthesis of hindered β-aminoesters. In the reactions carried out the β-aminoester is produced from methyl or ethyl crotonate as α, β- unsaturated compound and different amines. Besides other catalysts, Eu(fod)₃ and ytterbium triflate (Yb(OTf)_{3₎} are employed. Of the two lanthanide compounds only Ytterbium triflate improved the yield whereby as Table 2 of this document shows good yields are only achieved under high pressure.

Houben-Weyl Methoden der Organischen Chemie, Vol. XI/1, 267-293, 1957, mentions that the addition of a amines to unsaturated double-bonds is enhanced by strongly polarized groups such as α, β- unsaturated nitriles, carbonic acids and their derivatives, aldehydes, ketones, sulfones and nitro compounds.

On the other hand, S. Matsubara et al. reports in Chemistry Letters, 827 to 830 (1994) that Yb(OTf)₃, La(OTf)₃ and Sm(OTf)₃ accelerate the addition reaction of an amine compound to an α , *β*-unsaturated compound. However, no substance capable of accelerating the addition reaction of the amino compound to the α , β -unsaturated compound having a nitrile, carboxyl, sulfonyl, carbamoyl or nitro group was known in the prior art.

### Summary of the Invention

An object of the present invention is to provide a method for efficiently carrying out the addition reaction of an amine compound to an α , β -unsaturated compound having a specified substituent such as nitrile.

This and other objects of the present invention will be apparent from the following description and Examples.

The present invention has been completed on the basis of a finding that an inorganic salt or oxide of a lanthanoid effectively accelerates the addition reaction of the amine compound to the α , β-unsaturated compound having a specified substituent such as nitrile.

Namely, the present invention provides a method for producing an amine compound of the following general formula (I), which comprises the step of adding an amine compound (B) to an α , β -unsaturated compound (A) in the presence of an inorganic salt or oxide of a lanthanoid: 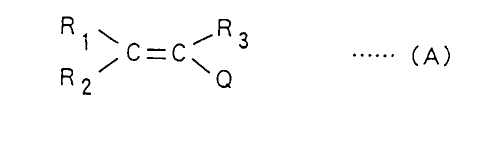 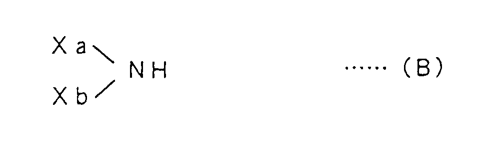 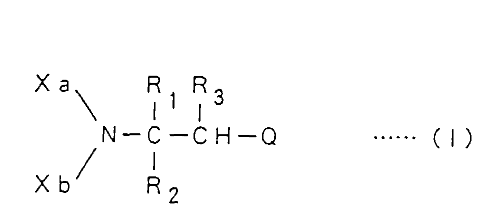 wherein R₁, R₂ and R₃ each represent a hydrogen atom or substituent, Q represents a nitrile group, carboxyl group, sulfonyl group, acyl group, carbamoyl group or nitro group, and Xa and Xb each represent a hydrogen atom or substituent.

The present invention also provides a method for producing a diamine compound of the following general formula (II), which comprises the step of adding a diamine (B²) to an α , β -unsaturated compound (A) in the presence of an inorganic salt or oxide of a lanthanoid: 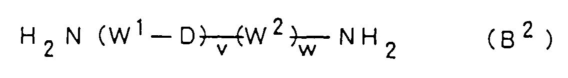 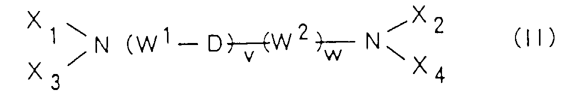 wherein Q, R₁, R₂ and R₃ are as defined above, W¹ and W² each represent an alkylene group, arylene group, aralkylene group or divalent nitrogen-containing heterocyclic group, D represents a single bond, -O-, -S- or -N(Rw)-, Rw being a hydrogen atom, aliphatic hydrocarbon group, aryl group or heterocyclic group, v represents an integer of 0 to 3, w represents an integer of 1 to 3, and X₁, X₂, X₃ and X₄ each represent a hydrogen atom or Xaaa, with the proviso that all of X₁, X₂, X₃ and X₄ cannot be hydrogen atom at the same time 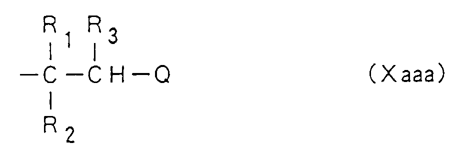 wherein Q, R₁, R₂ and R₃ are as defined above.

### Description of the Preferred Embodiments

The detailed description will be given on the method for producing the addition reaction product of the present invention.

The inorganic salts and/or oxides of lanthanoids are used in the present invention. They can be used either singly or in the form of a mixture of two or more of them. The inorganic salts of lanthanoids include, for example, chlorides, fluorides, iodides, bromides, nitrates, sulfates, carbonates and borides of them. Among these salts, the chlorides, nitrates, sulfates and carbonates are preferred. The chlorides are particularly preferred.

Examples of the lanthanoids include lanthanum, cerium, praseodymium, neodymium, promethium, samarium, europium, gadolinium, terbium, dysprosium, holmium, erbium, thulium, ytterbium and lutetium. Among them, preferred are lanthanum, cerium, neodymium, samarium, europium, gadolinium, terbium, dysprosium, holmium, erbium and ytterbium. Among them more preferred are lanthanum, cerium, neodymium, samarium and ytterbium. Lanthanum, cerium and ytterbium are particularly preferred.

The lanthanoid salts are preferably lanthanum chloride, cerium chloride, neodymium chloride, samarium chloride, europium chloride, gadolinium chloride, terbium chloride, dysprosium chloride, holmium chloride, erbium chloride, ytterbium chloride, lanthanum fluoride, lanthanum iodide, lanthanum bromide, lanthanum carbonate, cerium carbonate, neodymium carbonate, samarium carbonate, europium carbonate, gadolinium carbonate, terbium carbonate, dysprosium carbonate, holmium carbonate, erbium carbonate, ytterbium carbonate, lanthanum sulfate and lanthanum nitrate. Among them, more preferred are lanthanum chloride, cerium chloride, neodymium chloride, samarium chloride, ytterbium chloride, lanthanum carbonate, cerium carbonate, neodymium carbonate, samarium carbonate and ytterbium carbonate. Among them, still more preferred are lanthanum chloride, cerium chloride, ytterbium chloride, lanthanum carbonate and cerium carbonate. Particularly preferred are lanthanum chloride, cerium chloride and ytterbium chloride.

The lanthanoid oxides are preferably lanthanum oxide, cerium oxide, neodymium oxide, samarium oxide, europium oxide, gadolinium oxide, terbium oxide, dysprosium oxide, holmium oxide, erbium oxide and ytterbium oxide. Among them, more preferred are lanthanum oxide, cerium oxide, neodymium oxide, samarium oxide and ytterbium oxide. Among them, most preferred are lanthanium oxide and cerium oxide.

The amount of the inorganic salt and/or oxide of the lanthanoid is 0.1 to 100 molar %, preferably 0.1 to 10 molar %, more preferably 0.5 to 5 molar % and particularly preferably 0.5 to 2 molecular %, based on the α,β-unsaturated compound when a solvent other than water is used or when no solvent is used. It is preferably 1 to 200 molar %, more preferably 1 to 100 molar % and most preferably 5 to 50 molar % when water is used as the solvent. Particularly preferred amount is 10 to 50 molar %.

Although the timing of the addition of the inorganic salt and/or oxide of the lanthanoid is not particularly limited, it is added preferably in the period ranging from the feeding of the starting materials to the initial stage of the reaction. Although the form of the inorganic salt and/or oxide to be added is also not particularly limited, the surface area thereof is preferably as large as possible. As to the manner of the addition of the inorganic salt and/or oxide, it may be added in the form or a solution or suspension in a solvent or the starting material or, alternatively, it may be directly added as it is. The direct addition is preferred. The inorganic acid and/or oxide can be repeatedly used, and it can be recovered by the filtration after the completion of the reaction.

The reaction temperature is preferably 10 to 200°C , more preferably 50 to 200 °C and most preferably 70 to 170°C. The reaction temperature is preferably as high as possible so far as the starting materials and the reaction product are not decomposed.

The reaction proceeds in a reaction solvent or in the absence of the solvent. When the solvent is used, it may be any solvent inert to the reaction. The solvents include, for example, alcohols (such as methanol, ethanol, 2-propanol and butanol), ethers (such as dioxane and tetrahydrofuran), nitriles (such as acetonitrile), amides (such as dimethylformamide and dimethylacetamide), hydrocarbons (such as hexane), halogenated hydrocarbons (such as chloroform and dichloromethane) and water. Preferably the reaction is carried out in the absence of the solvent.

The reaction is carried out preferably under a weakly acidic to strongly basic condition, more preferably under neutral to strongly basic condition. Although a base is preferably added to the reaction system, the reaction can be conducted without the base. When the reaction system is made basic by the starting amine, the addition of the base can be omitted.

The amount of the α,β-unsaturated compound (A) to be added to the amino group-containing compound is preferably 0.5 to 4 mols per mol of the amino group when one compound (A) is to be added to one amino group. However, it is more preferably 0.8 to 3 mols and most preferably 0.9 to 2 mols pre mol of the amino group. When two compound (A) is to be added to one amino group, 1 to 4 mols, preferably 1.5 to 3.5 mols and more preferably 1.8 to 3 mols, of the compound (A) is added to one mol of the amino group.

The detailed description will be given on the α,β-unsaturated compound (A) used in the present invention.

R₁, R₂ and R₃ in the above formulae each represent a hydrogen atom or substituent. The substituents represented by R₁, R₂ and R₃ include aliphatic hydrocarbon groups, aryl groups, amino groups (those having preferably 0 to 20 carbon atoms, more preferably 0 to 10 carbon atoms and particularly preferably 0 to 6 carbon atoms, such as amino, methylamino, dimethylamino and diethylamino groups), alkoxy groups (those having preferably 1 to 8 carbon atoms, more preferably 1 to 6 carbon atoms and particularly preferably 1 to 4 carbon atoms, such as methoxy and ethoxy groups), aryloxy groups (those having preferably 6 to 12 carbon atoms, more preferably 6 to 10 carbon atoms and particularly preferably 6 to 8 carbon atoms, such as phenyloxy group), acyl groups (those having preferably 1 to 12 carbon atoms, more preferably 2 to 10 carbon atoms and particularly preferably 2 to 8 carbon atoms, such as acetyl group), alkoxycarbonyl groups (those having preferably 2 to 12 carbon atoms, more preferably 2 to 10 carbon atoms and particularly preferably 2 to 8 carbon atoms, such as methoxycarbonyl group), aryloxycarbonyl groups (those having preferably 7 to 20 carbon atoms, more preferably 7 to 15 carbon atoms and particularly preferably 7 to 10 carbon atoms, such as phenyloxycarbonyl group), acyloxy groups (those having preferably 2 to 12 carbon atoms, more preferably 2 to 10 carbon atoms and particularly preferably 2 to 8 carbon atoms, such as acetoxy group), acylamino groups (those having prefrably 2 to 10 carbon atoms, more preferably 2 to 6 carbon atoms and particularly preferably 2 to 4 carbon atoms, such as acetylamino group), alkoxycarbonylamino groups (those having preferably 2 to 12 carbon atoms, more preferably 2 to 10 carbon atoms and particularly preferably 2 to 8 carbon atoms, such as methoxycarbonylamino group), aryloxycarbonylamino groups (those having preferably 7 to 20 carbon atoms, more preferably 7 to 12 carbon atoms and particularly preferably 7 to 10 carbon atoms, such as phenyloxycarbonylamino group), sulfonylamino groups (those having preferably 1 to 10 carbon atoms, more preferably 1 to 6 carbon atoms and particularly preferably 1 to 4 carbon atoms, such as methanesulfonylamino group), sulfamoyl groups (those having preferably 0 to 10 carbon atoms, more preferably 0 to 6 carbon atoms and particularly preferably 0 to 4 carbon atoms, such as sulfamoyl and methylsulfamoyl groups), carbamoyl groups (those having preferably 1 to 10 carbon atoms, more preferably 1 to 6 carbon atoms and particularly preferably 1 to 4 carbon atoms, such as carbamoyl and methylcarbamoyl groups), alkylthio groups (those having preferably 1 to 8 carbon atoms, more preferably 1 to 6 carbon atoms and particularly preferably 1 to 4 carbon atoms, such as methylthio, ethylthio and carboxymethylthio groups), arylthio groups (those having preferably 6 to 20 carbon atoms, more preferably 6 to 10 carbon atoms and particularly preferably 6 to 8 carbon atoms, such as phenylthio group), sulfonyl groups (those having preferably 1 to 8 carbon atoms, more preferably 1 to 6 carbon atoms and particularly preferably 1 to 4 carbon atoms, such as methanesulfonyl group), sulfinyl groups (those having preferably 1 to 8 carbon atoms, more preferably 1 to 6 carbon atoms and particularly preferably 1 to 4 carbon atoms, such as methanesulfinyl group), ureido groups (those having preferably 1 to 8 carbon atoms, more preferably 1 to 6 carbon atoms and particularly preferably 1 to 4 carbon atoms, such as ureido and methylureido groups), hydroxy group, mercapto group, halogen atoms (such as fluorine, chlorine, bromine and iodine atoms), cyano group, sulfo group, carboxyl group, nitro group, hydroxamic acid group and heterocyclic groups. These substituents may be further substituted with a carboxyl group, alkyl group, hydroxyl group, acyl group or the like.

The aliphatic hydrocarbon groups represented by R₁, R₂ and R₃ include, for example, linear, branched or cyclic alkyl groups (those having preferably 1 to 12 carbon atoms, more preferably 1 to 7 carbon atoms and particularly preferably 1 to 4 carbon atoms, such as methyl, ethyl, n-propyl, iso-propyl, n-butyl, iso-butyl, tert-butyl, n-heptyl, n-hexyl and cyclohexyl groups), alkenyl groups (those having preferably 2 to 12 carbon atoms, more preferably 2 to 6 carbon atoms and particularly preferably 2 to 4 carbon atoms, such as vinyl and allyl groups), and alkynyl groups (those having preferably 2 to 12 carbon atoms, more preferably 2 to 6 carbon atoms and particularly preferably 2 to 4 carbon atoms, such as propargyl group). Among them, the alkyl groups having 1 to 12 carbon atoms are preferred, those having 1 to 7 carbon atoms are more preferred, and those having 1 to 4 carbon atoms are particularly preferred.

The aliphatic hydrocarbon groups are preferably methyl, ethyl, n-propyl, iso-propyl, n-butyl, benzyl, hydroxymethyl, carboxymethyl and 5-imidazolylmethyl groups. Among them, methyl, ethyl, n-propyl, iso-propyl and n-butyl groups are particularly preferred.

The aryl groups represented by R₁, R₂ and R₃ may be a monocyclic ring or they may form a condensed ring with another ring. They have preferably 6 to 20 carbon atoms, more prefrably 6 to 16 carbon atoms and most preferably 6 to 12 carbon atoms. The aryl group is preferably a monocyclic or bicyclic aryl group such as phenyl or naphthyl group. The phenyl group is more preferred.

The heterocyclic groups represented by R₁, R₂ and R₃ are 3- to 10-membered, saturated or unsaturated heterocyclic groups containing at least one of N, O and S atoms. The heterocyclic group may be monocyclic or may form a condensed ring with another ring. The heterocyclic group is preferably a 5-membered or 6-membered aromatic heterocyclic ring, more preferably that containing a nitrogen atom, and most preferably that containing one or two nitrogen atoms.

Examples of the heterocyclic groups include pyrrolidyl, piperidino, piperidyl, piperadyl, morpholino, morpholinyl, thienyl, furyl, pyrrolyl, imidazolyl, pyrazolyl, pyridyl, pyradyl, pyridazyl, triazolyl, triazyl, indolyl, indazolyl, purinyl, thiadiazolyl, oxadiazolyl, quinolinyl, phthalazyl, naphthyridyl, quinoxalyl, quinazolyl, cinnolyl, pteridyl, acridinyl, phenanthrolyl, phenazinyl, tetrazolyl, thiazolyl and oxazolyl groups. The preferred heterocyclic groups are pyrrolyl, imidazolyl, pyrazolyl, pyridyl, pyradyl, pyridazyl, triazolyl, triazyl, indolyl, indazolyl, thiadiazolyl, oxadiazolyl, quinolinyl, phthalazyl, quinoxalyl, quinazolyl, cinnolyl, tetrazolyl, thiazolyl and oxazolyl groups. Particularly preferred are imidazolyl, pyrazolyl, pyridyl, pyradyl, indolyl, indazolyl, thiadiazolyl, oxadiazolyl, quinolinyl, thiazolyl and oxazolyl groups. More particularly preferred are imidazolyl, pyridyl and quinolinyl groups. The most preferred are imidazolyl and pyridyl groups.

The substituents represented by R₁, R₂ and R₃ are preferably alkyl, aryl, alkoxy, carboxyl, hydroxy, heterocyclic, nitrile and carbamoyl groups. Among them, the alkyl, aryl, carboxyl and hydroxy groups are particularly preferred. Among them, the alkyl, aryl and carboxyl groups are more particularly preferred. The alkyl group is particularly preferred.

R₁, R₂, R₃ and Q may be combined with one another to form a ring, if possible.

Either R₁ or R₂ is preferably a hydrogen atom. When either R₁ or R₂ is a substituent, R₃ is preferably a hydrogen atom.

Q represents a nitrile, carboxyl, sulfonyl, acyl, carbamoyl or nitro group. In such a group, nitrile, carboxyl or acyl group is preferred. Nitrile or carboxyl group is more preferred. Nitrile group is particularly preferred.

Among the compounds (A), compounds (A₁) are preferred, and compounds (A₁₁) are more preferred. 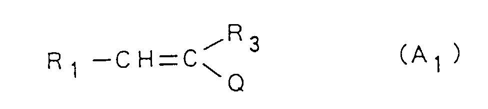 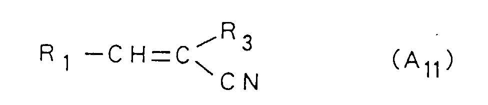 wherein R₁, R₃ and Q are as defined above for the compounds (A).

The detailed description will be made on the amine compounds (B) used in the present invention.

The substituents represented by Xa and Xb are those listed above as the substituents R₁, R₂ and R₃. Among these substituents, preferred substituents are aliphatic hydrocarbon groups [such as linear, branched or cyclic alkyl groups (those having preferably 1 to 12 carbon atoms, more preferably 1 to 7 carbon atoms and particularly preferably 1 to 4 carbon atoms, such as methyl, ethyl, n-propyl, iso-propyl, n-butyl, iso-butyl, tert-butyl, n-heptyl, n-hexyl and cyclohexyl groups)], aryl groups (monocyclic aryl groups and those forming a condensed ring with another ring, which have preferably 6 to 20 carbon atoms, more preferably 6 to 16 carbon atoms and particularly preferably 6 to 12 carbon atoms; preferred examples of them being monocyclic or bicyclic aryl groups such as phenyl and naphthyl groups; phenyl group being particularly preferred; the aryls group being substituted or unsubstituted), amino groups (those having preferably 0 to 20 carbon atoms, more preferably 0 to 10 carbon atoms and particularly preferably 0 to 6 carbon atoms, such as amino, methylamino, dimethylamino and diethylamino groups), acyl groups (those having preferably 1 to 12 carbon atoms, more preferably 2 to 10 carbon atoms and particularly preferably 2 to 8 carbon atoms, such as acetyl group), sulfonyl groups (those having preferably 1 to 8 carbon atoms, more preferably 1 to 6 carbon atoms and particularly preferably 1 to 4 carbon atoms, such as methanesulfonyl group), hydroxy group, and heterocyclic groups (3- to 10-membered, saturated or unsaturated heterocyclic groups having at least one of N, O and S atoms, which may be monocyclic heterocyclic groups or may form a condensed ring together with another ring; the heterocyclic rings being preferably 5- or 6-membered aromatic heterocyclic rings, more preferably 5-membered or 6-membered aromatic heterocyclic rings containing a nitrogen atom, and most preferably 5- or 6-membered aromatic heterocyclic ring containing 1 or 2 nitrogen atoms; examples of the heterocyclic groups are those listed above as examples of the heterocyclic groups represented by R₁, R₂ and R₃). These substituents may be further substituted.

Xa is preferably an aliphatic hydrocarbon group, aryl group, hydroxy group or heterocyclic group. Among them, the alkyl or aryl group is more preferred, and the alkyl group is particularly preferred.

Xb is preferably a hydrogen atom or aliphatic hydrocarbon group, in particular a hydrogen atom or alkyl group. Particularly preferred is a hydrogen atom or a group represented by Xaaa: 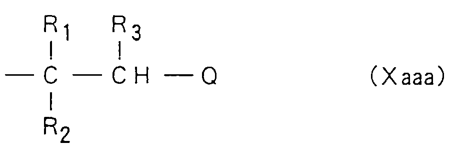 wherein R₁, R₂, R₃ and Q are as defined above.

Xa and Xb may form a ring. The ring is preferably 4- to 6-membered ring, more preferably 5- or 6-membered ring. Among them, the 6-membered ring is preferred. The ring may have a substituent which is the same as that referred to above as the substituent of R₁, R₂ and R₃. The ring may form a condensed ring with another ring.

The amine compounds (B) are preferably amines (B₁ₐ), more preferably amines (B_{1b}) and particularly amines (B_{1c}). 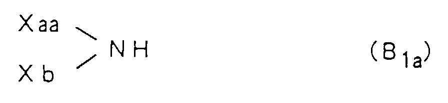 wherein Xb is as defined above for the amine compounds (B) and the preferred range thereof is also the same as that described above, and Xaa represents an alkyl group, aryl group or heterocyclic group and the preferred range thereof is the same as that described above for Xa). 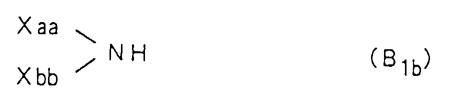 wherein Xaa is as defined above for the amine compounds (B₁ₐ) and the preferred range thereof is also the same as that described above, and Xbb represents a hydrogen atom or a group represented by the following formula Xaaa: 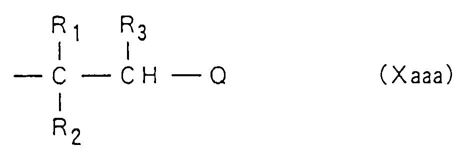 wherein R₁, R₂, R₃ and Q are as defined above.

Xaa-NH₂ (B_{1c})

wherein Xaa is as defined above for the amine compounds (B₁ₐ) and the preferred range thereof is also the same as that described above.

Among the amines (B_{1c} ), preferred are aliphatic amines having 1 to 20 carbon atoms, particularly 1 to 15 carbon atoms. More preferred are aliphatic diamines having 2 to 15 carbon atoms. Among the aliphatic diamines, diamines (B²) of the following formula are preferred: 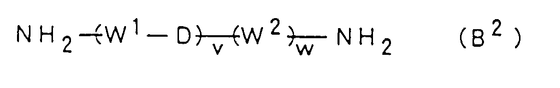 wherein W¹ and W² each represent an alkylene group, arylene group, aralkylene group or divalent nitrogen-containing heterocyclic group, D represents a single bond, -O-, -S- or -N(Rw)-, Rw being a hydrogen atom, aliphatic hydrocarbon group, aryl group or heterocyclic group, v represents an integer of 0 to 3, and w represents an integer of 1 to 3.

The alkylene groups represented by W¹ and W² may be linear, branched or cyclic. The linear or branched alkylene groups are preferred. The number of carbon atoms forming the alkylene group is preferably 2 to 8, more preferably 2 to 6 and particularly preferably 2 to 4. The alkylene groups represented by W¹ and W² include, for example, ethylene, propylene, trimethylene, tetramethylene and 1,2-cyclohexylene groups. Among them, ethylene, propylene, trimethylene and tetramethylene groups are preferred, and ethylene and trimethylene groups are particularly preferred.

The arylene groups represented by W¹ and W² may be monocyclic arylene groups or may form a condensed ring together with another ring. They have preferably 6 to 20 carbon atoms, more preferably 6 to 16 carbon atoms and most preferably 6 to 12 carbon atoms.

The arylene groups represented by W¹ and W² are preferably monocyclic or bicyclic arylene groups such as phenylene and naphthylene groups. Among them, phenylene group is preferred.

The aralkylene groups represented by W¹ and W² are those having preferably 7 to 21 carbon atoms, more preferably 7 to 17 carbon atoms and still preferably 7 to 13 carbon atoms, such as o-xylenyl group.

The divalent, nitrogen-containing heterocyclic groups represented by W¹ and W² are 5-membered or 6-membered heterocyclic groups in which the hetero atom is nitrogen, such as imidazolyl group.

W¹ and W² may have a substituent. The substituents are the same as those of the α,β-unsaturated compounds (A), and the preferred range of them is the same as that of the compounds (A).

D represents a single bond, -O-, -S- or -N(Rw)-. The aliphatic hydrocarbon groups, aryl groups and heterocyclic groups represented by Rw are the same as those represented by R₁. The aliphatic hydrocarbon groups, aryl groups and heterocyclic groups represented by Rw may have a substituent. The substituents are the same as those of the α,β-unsaturated compounds (A), and preferred range of them is the same as that of the compounds (A).

The substituents of Rw are preferably carboxyl, phosphono, hydroxy and sulfo groups. Among them, carboxyl group is more preferred.

Rw is preferably a substituted or unsubstituted alkyl group having 1 to 8 carbon atoms (such as methyl or carboxymethyl group), aryl group having 6 to 10 carbon atoms (such as phenyl group) or heterocyclic group having 1 to 9 carbon atoms (such as pyridyl group).

v represents an integer of 0 to 3, and when v is 2 or 3, (W¹-D)'s may be the same or different from each other. v is preferably 0 to 2, more preferably 0 or 1, and particularly preferably 0. w represents an integer of 1 to 3. When w is 2 or 3, W²'s may be the same or different from each other. w is preferably 1 or 2.

Examples of -(W¹-D) ᵥ -(W²) _{w} - include those shown below:

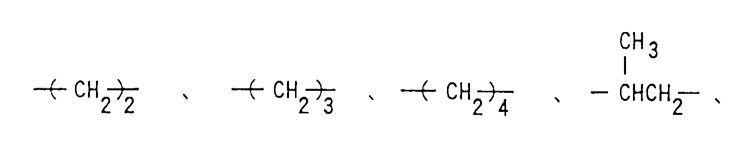

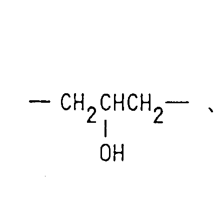

- CH₂CH₂OCH₂CH₂- ,

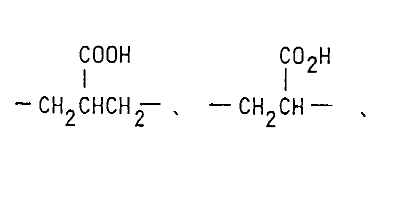

- CH₂CH₂OCH₂CH₂OCH₂CH₂- ,

- CH₂CH₂SCH₂CH₂- ,

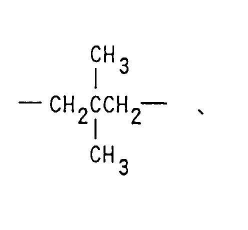

- CH₂CH₂SCH₂CH₂SCH₂CH₂- ,

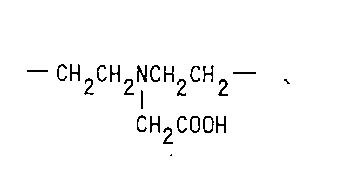

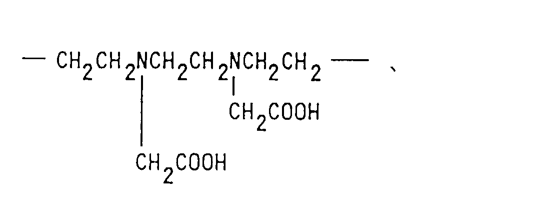

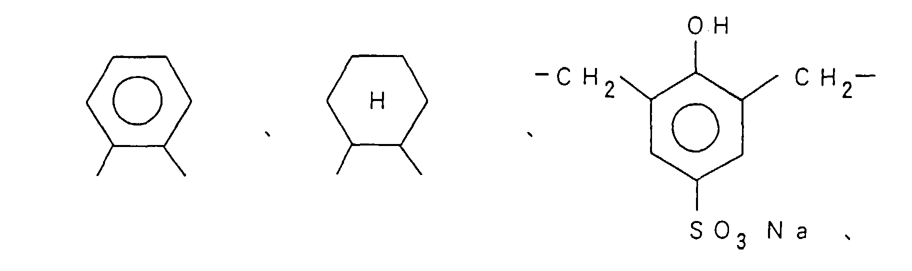

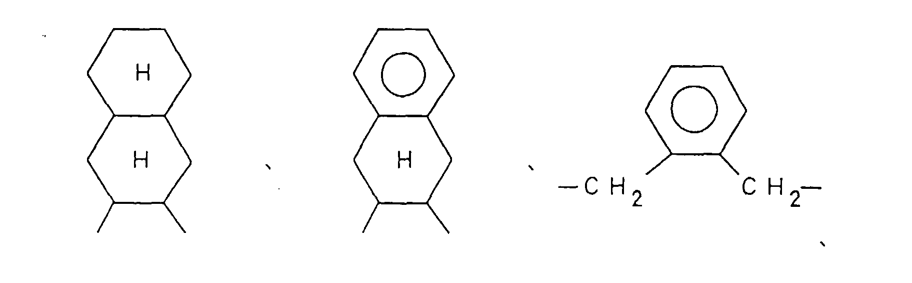

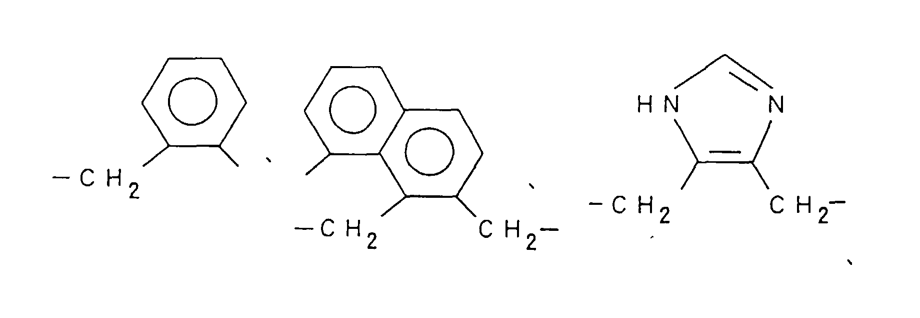

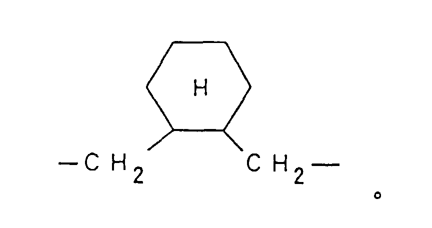

-(W¹-D) ᵥ -(W²) _{w} - are preferably alkylene groups.

The alkylene groups represented by W may be linear, branched or cyclic alkylene groups, preferably linear or branched alkylene groups. The number of carbon atoms forming the alkylene group is preferably 2 to 8, more preferably 2 to 6 and particularly preferably 2 to 4. The alkylene groups represented by W include, for example, ethylene, propylene, trimethylene, tetramethylene and 1,2-cyclohexylene groups. Among them, preferred are ethylene, propylene, trimethylene and tetramethylene groups, and ethylene and trimethylene groups are more preferred.

Among the diamines (B²), preferred are diamines (B^{2a}) of the following formula:

NH₂-W-NH₂ (B^{2a})

wherein W represents an alkylene group.

The detailed description will be given on the compounds of the general formulae (I) and (II) which can be synthesized by the method of the present invention.

R₁, R₂ and R₃ in the general formulae (I) and (II) are the same as those of the α,β-unsaturated compounds (A), and preferred ranges of them are the same as those of the compounds (A). Further the substituents represented by Xa and Xb are the same as those of the amine compounds (B), and preferred ranges of them are also the same as those of the amine compounds (B). W¹, W², D, v and w of the compounds represented by the general formula (II) are the same as those of the diamines (B²), and preferred ranges of them are the same as those of the diamines (B²).

Among the compounds of the general formula (I), prefrred are those of the following general formula (III): 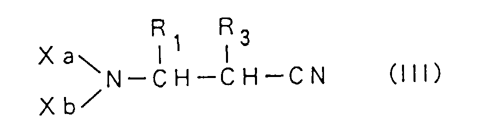 wherein R₁, R₃, Xa and Xb are as defined above for the general formula (I), and preferred ranges of them are the same as those of the general formula (I).

Among the compounds represented by the general formula (II), preferred are those of the following general formula (IV), and more preferred are those of the following general formula (V): 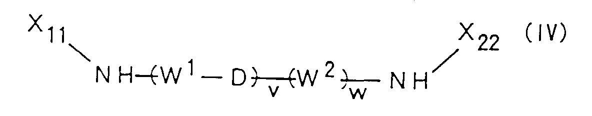 wherein W¹, W², D, v and w are the same as those of the general formula (II), and preferred ranges of them are also the same as those of the general formula (II), X₁₁ and X₂₂ are the same as X₁ and X₂, respectively, in the general formula (II), with the proviso that both X₁₁ and X₂₂ cannot be hydrogen atom at the same time, and 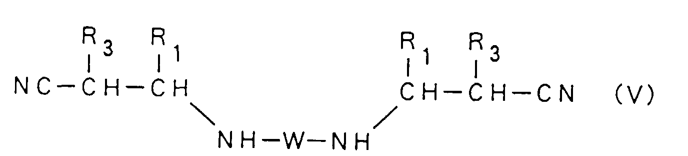 wherein R₁ and R₃ are as defined in the general formula (II), and preferred ranges of them are the same as those of the general formula (II), and W is as defined with reference to the diamines (B^{2a}), and the preferred ranges thereof is the same as that of the diamines (B^{2a}).

Examples of the compounds of the general formula (I) which can be synthesized by the method of the present invention will be given below, which by no means limit the invention.

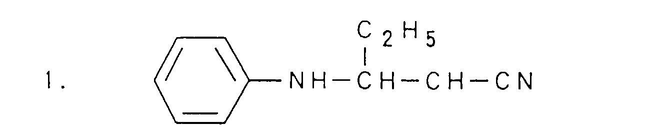

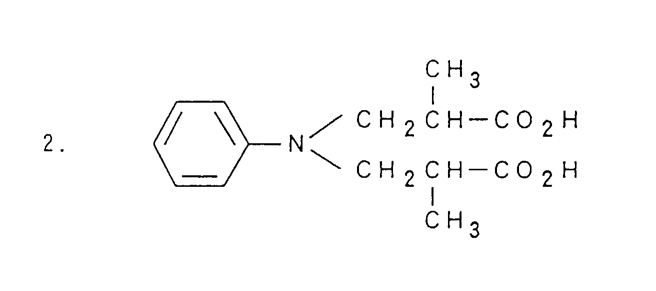

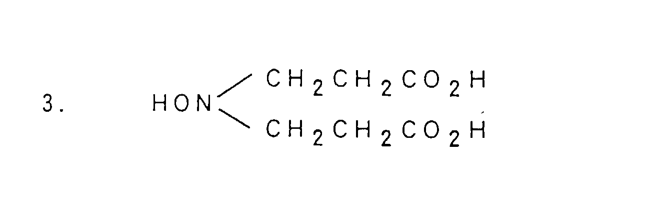

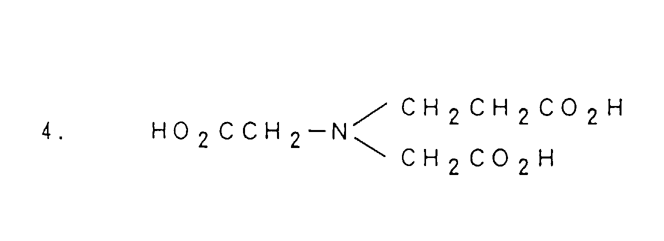

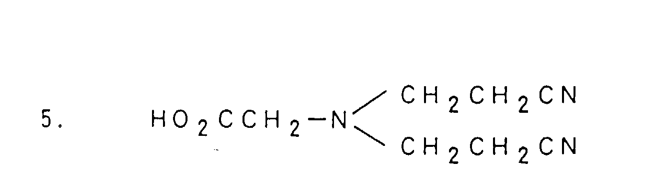

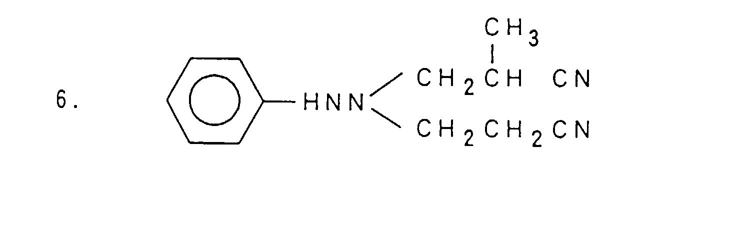

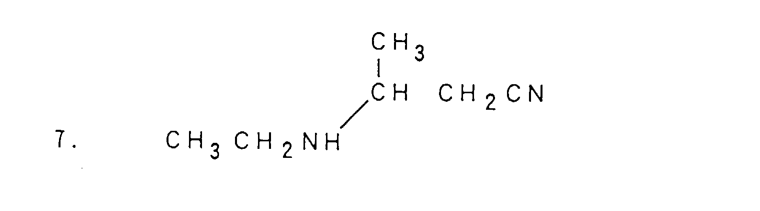

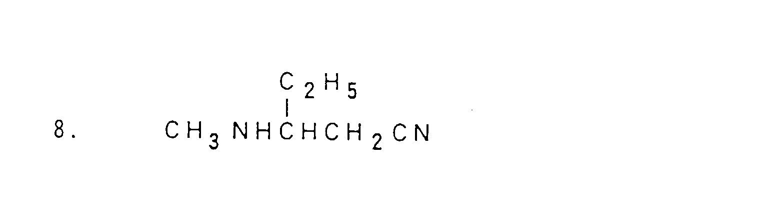

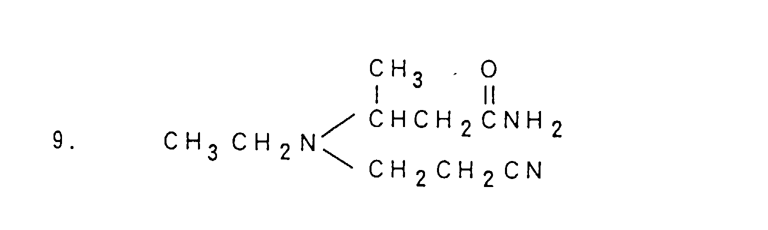

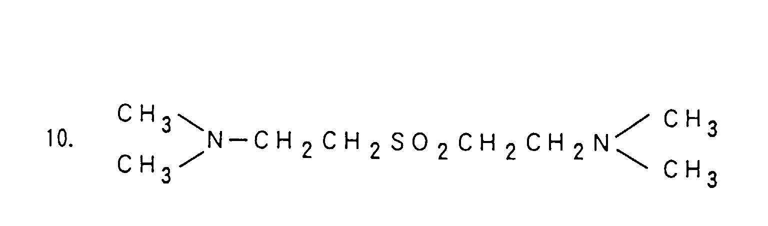

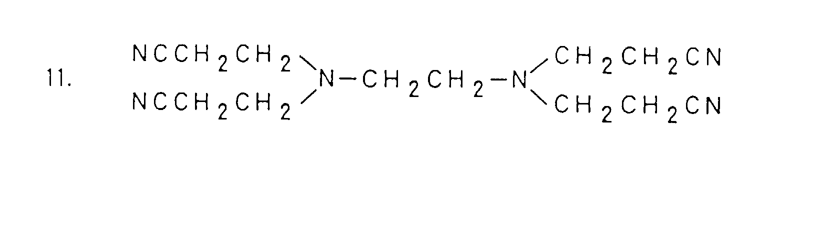

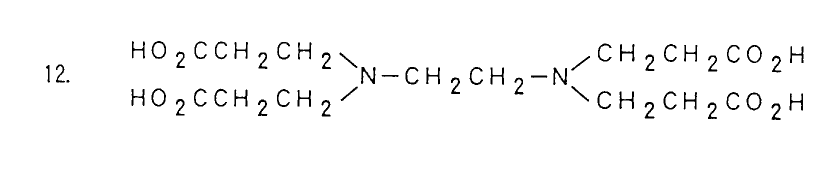

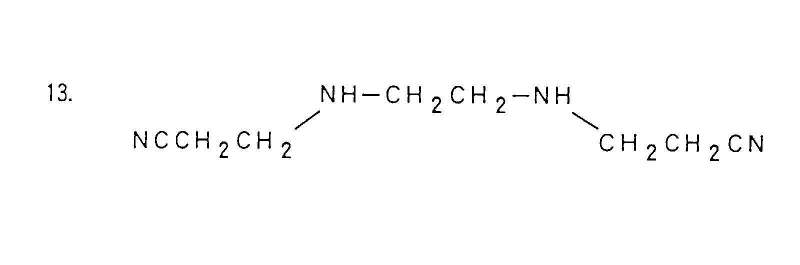

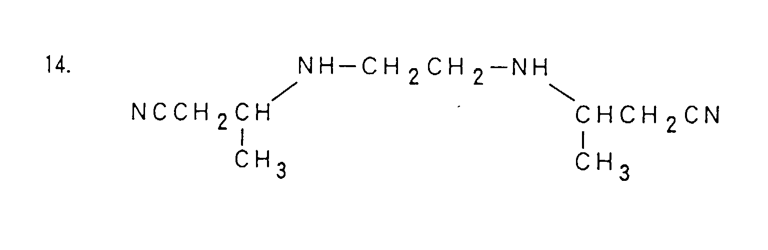

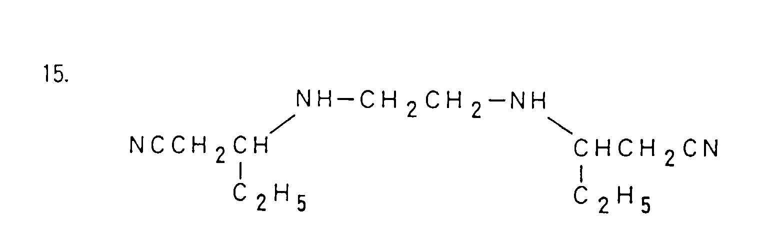

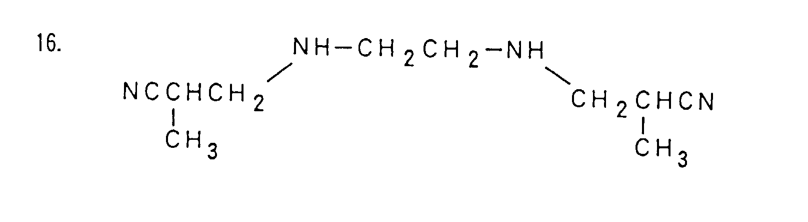

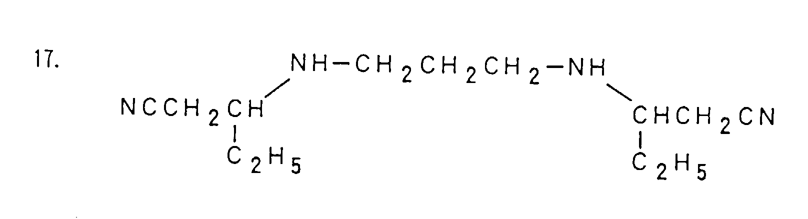

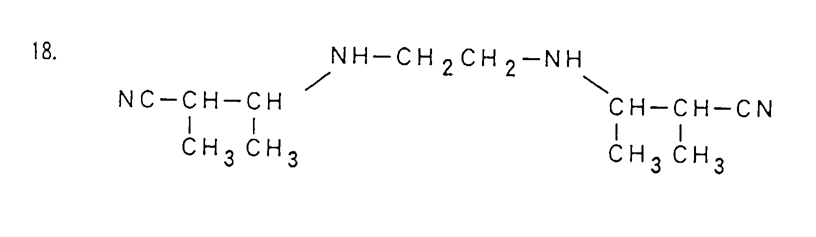

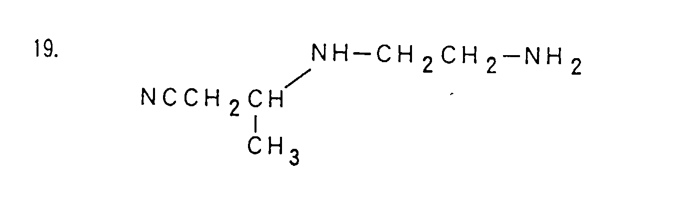

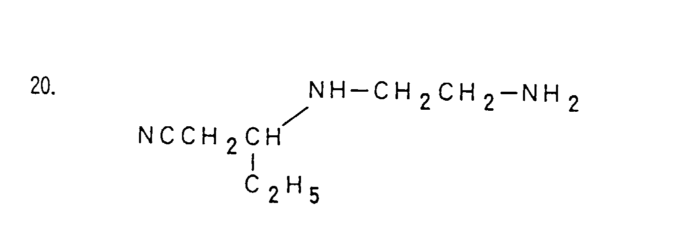

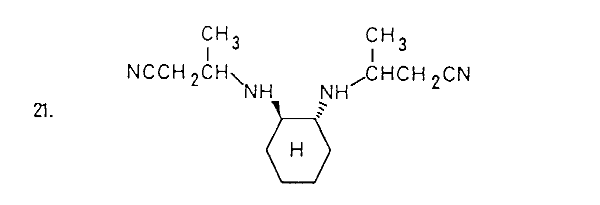

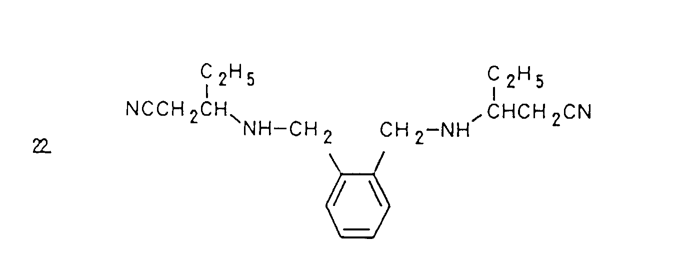

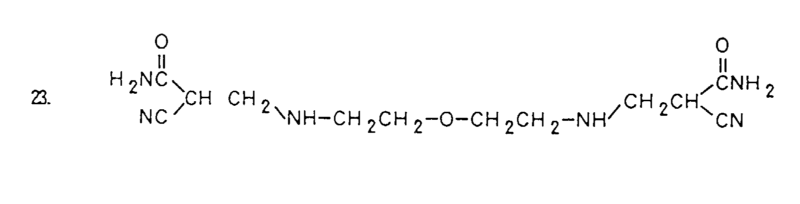

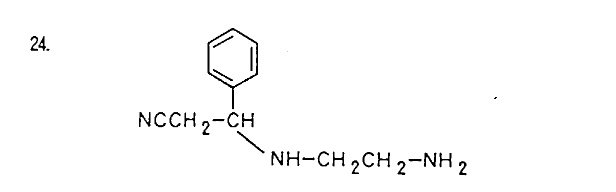

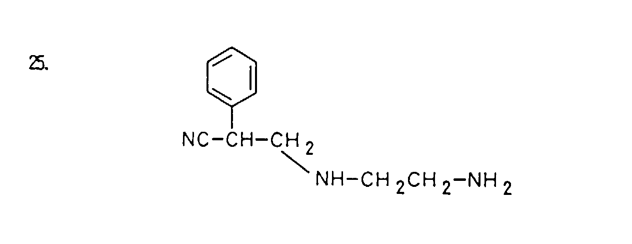

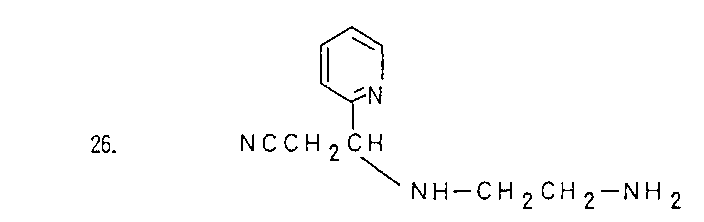

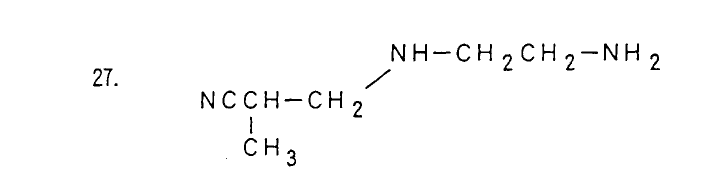

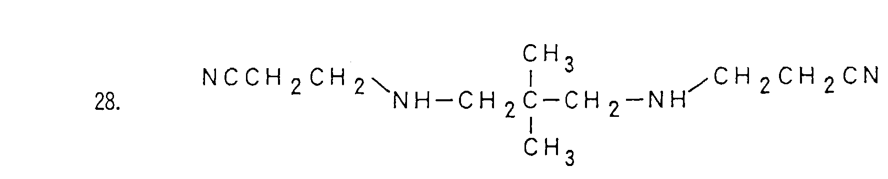

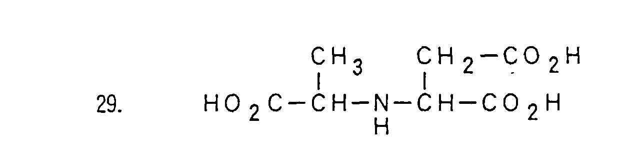

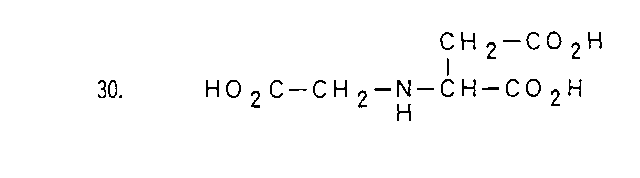

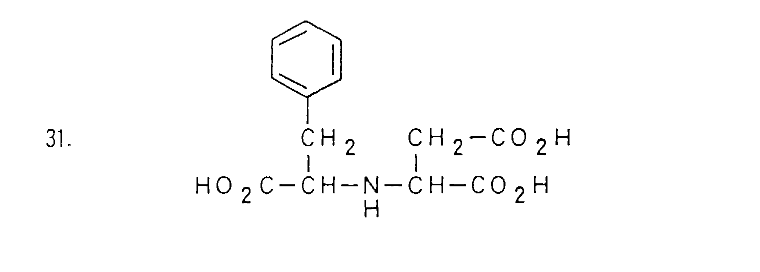

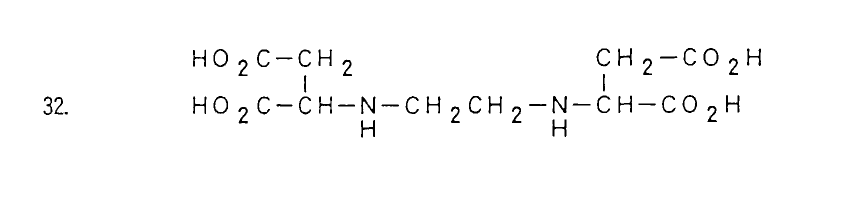

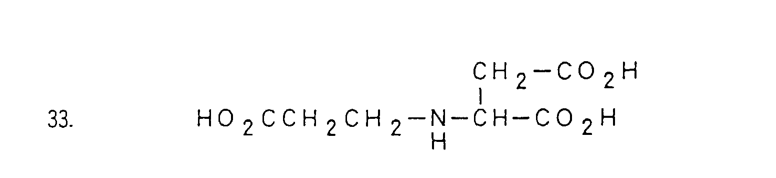

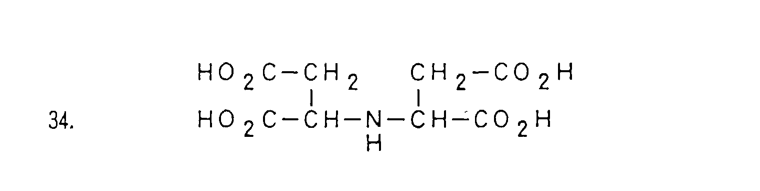

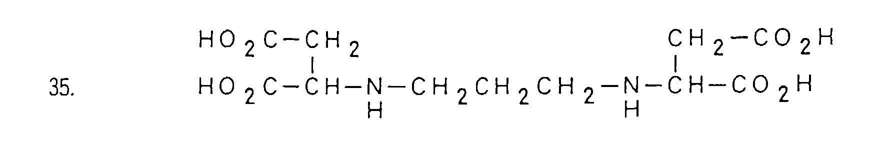

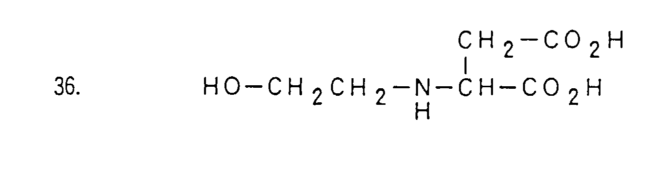

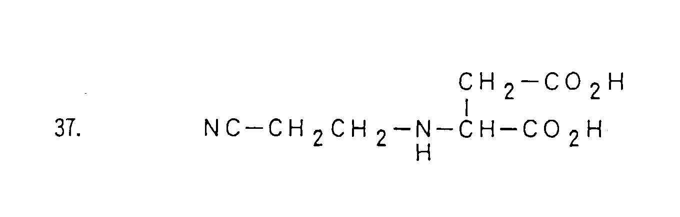

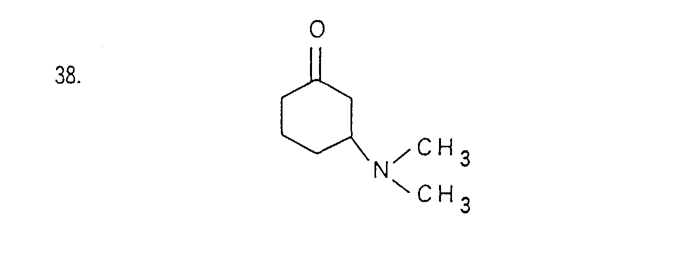

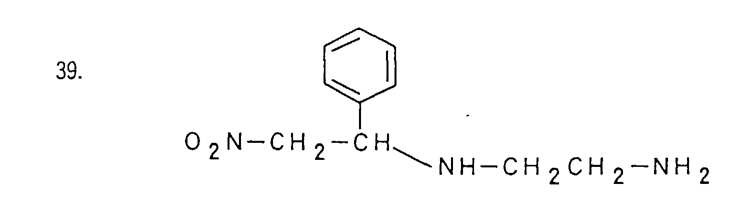

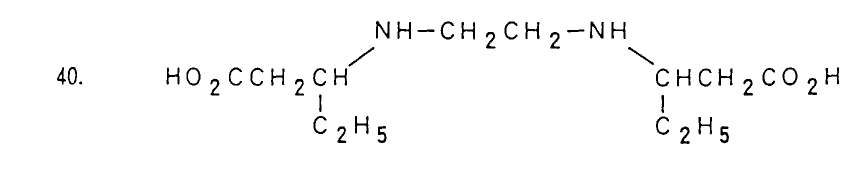

The description given above relates to the preferred ranges of those usable in the present invention. Preferred combinations of them are as given below. The larger the number, the more preferred.
1. A method wherein an amine compound of the general formula (I) is produced from an amine compound (B) and an α,β-unsaturated compound (A) in the presence of an inorganic salt or oxide of a lanthanoid at a reaction temperature of 50 to 200°C.
2. A method wherein an amine compound of the general formula (II) is produced from a diamine (B²) and an α,β-unsaturated compound (A) in the presence of an inorganic salt or oxide of a lanthanoid at a reaction temperature of 50 to 200°C .
3. A method wherein an amine compound of the general formula (IV) is produced from a diamine (B²) and an α,β-unsaturated compound (A) in the presence of an inorganic salt or oxide of a lanthanoid at a reaction temperature of 50 to 200°C .
4. A method according to the above method 3, wherein the α,β-unsaturated compound (A) is an α,β-unsaturated compound (A₁).
5. A method wherein a compound of the general formula (V) is produced from a diamine (B^{2a}) and an α,β-unsaturated nitrile (A₁₁) in the presence of an inorganic salt or oxide of a lanthanoid at a reaction temperature of 50 to 200 °C.
6. A method wherein a compound of the general formula (V) is produced from a diamine (B^{2a}) and an α,β-unsaturated nitrile (A₁₁) in the presence of 10 molar % or less, based on the α,β-unsaturated nitrile (A₁₁), of an inorganic salt or oxide of a lanthanoid at a reaction temperature of 50 to 200°C .
7. A method wherein a compound of the general formula (V) is produced by reacting a diamine (B^{2a}) with an α,β-unsaturated nitrile (A₁₁) in the presence of 10 molar % or less, based on the α,β-unsaturated nitrile (A₁₁), of an inorganic salt or oxide of a lanthanoid without using any solvent at a reaction temperature of 50 to 200°C.

When the reaction temperature is not below 50 °C, a remarkable effect of accelerating the reaction of the α,β-unsaturated compound used in the present invention is obtained unlike that of an α,β-unsaturated ester. Such an effect obtained by using the inorganic salt or oxide of the lanthanoid is more remarkable than that obtained by using a trifluoromethanesulfonate.

The effect of accelerating the reaction becomes more remarable when a diamine compound is used as the amine compound.

The following Examples will further illustrate the present invention.

### [Examples]

### Example 1

### (Synthesis of standard sample of compound 15)

100 g (1.66 mol) of ethylenediamine and 406 g (5.00 mol) of cis-2-pentenenitrile were fed into a three-necked flask, and heated under reflux for 20 hours. Then the reaction solution was distilled under reduced pressure (0.3 mmHg, 160 to 180 °C) with a vacuum pump to obtain 207 g (0.93 mol) of the intended compound 15. Yield: 56 %. The structure was confirmed by NMR.
1H NMR (CDCl₃, internal standard TMS)
δ 0.98 (t 6H)
δ 1.4-1.7 (m 6H)
δ 2.3-2.6 (m 4H)
δ 2.6-2.9 (m 6H)

### (Evaluation of reaction acceleration effect)

7.0 g (0.12 mol) of ethylenediamine, 28.3 g (0.35 mol) of cis-2-pentenenitrile and an inorganic salt of a lanthanoid (present invention) or an additive usually used for the Michael reaction in the prior art (comparative example) were fed into a 50 ml short-neck flask, and heated under reflux in an oil bath. Three hours after, the quantity of the intended product in the reaction solution was determined by high-performance liquid chromatography. The results are shown in Table 1.

**Table 1**

| Run No. | Additive | Yield of compound 15 after 3 h²⁾ (%) | Yield obtained by using recovred salt (%) | Remarks |
|---|---|---|---|---|
| 1 | none | not higher than 6 % | | Comp. Ex. |
| 2 | SnCl₄ | ditto | | ditto |
| 3 | TiCl₄ | ditto | | ditto |
| 4 | Triton B | undeterminable due to polymerization | | ditto |
| 5 | Neutral Al₂O₃ | 15 | | ditto |
| 6 | LaCl₃ · 7H₂O | 89 | 86 | present invention |
| 7 | CeCl₃ · 6H₂O | 87 | 83 | ditto |
| 8 | NdCl₃ · 6H₂O | 80 | 76 | ditto |
| 9 | SmCl₃ · 6H₂O | 81 | 76 | ditto |
| 10 | EuCl₃ · 6H₂O | 66 | 61 | ditto |
| 11 | GdCl₃ · 6H₂O | 72 | 69 | ditto |
| 12 | TbCl₃ · nH₂O¹⁾ | 74 | 70 | ditto |
| 13 | DyCl₃ · 6H₂O | 73 | 71 | ditto |
| 14 | HoCl₃ · 6H₂O | 78 | 75 | ditto |
| 15 | ErCl₃ · 6H₂O | 78 | 74 | ditto |
| 16 | YbCl₃ · 6H₂O | 85 | 83 | ditto |

| | | | | |
|---|---|---|---|---|
| ¹⁾ For convenience's sake, the amount was decided supposing n=6. | | | | |
| ²⁾ The yield based on ethylenediamine. | | | | |

When the lanthanoid salt was used, the precipitate in the reaction solution was recovered by filtration and the reaction was carried out again in the presence of the recovered precipitate in place of the lanthanoid salt under the above-described conditions. The results are also shown in Table 1.

It is understood from Table 1 that the lanthanoid salts have extremely excellent effect of accelerating the reaction and also that when the salts are recovered and repeatedly used, the excellent acceleration effect can be obtained.

### (Example wherein the lanthanoid salt was used)

The reaction solution, from which the lanthanoid salt had not been filtered out, obtained under the same conditions as those of Run No.6 was washed with a dilute aqueous sodium hydroxide solution. After extraction with ethyl acetate, the extract was dried over sodium sulfate. After filtration, ethyl acetate was distilled off under reduced pressure with an aspirator. Then the remaining reaction mixture was distilled under reduced pressure (0.3 mmHg, 160 to 180 °C) with a vacuum pump to obtain 21.6 g of the intended compound 15. Yield: 81 %.

### (Example wherein the subsequent reaction was carried out without the isolation)

The compound 15 obtained in Run No. 6 could be continuously subjected to the subsequent reaction without being isolated. The following Example shows the conversion reaction from the nitrile compound into the carboxyl compound.

### <Example wherein an acid is used ― synthesis of compound 40 from compound 15>

The whole reaction solution obtained in Run No. 6, from which the lanthanoid salt had not been filtered out, was slowly dropped into 100 g (0.96 mol) of cooled, concentrated hydrochloric acid so that the temperature could be kept not higher than 20°C. After the completion of the addition, the reaction mixture was heated under reflux in an oil bath for 3 hours and then cooled. The pH of the reaction solution was adjusted to 2 with 50 % aqueous sodium hydroxide solution. After desalting by the electrodialysis, the reaction solution was concentrated under reduced pressure. After leaving the concentrate to stand in a refrigerator for one week, the crystals thus formed were taken by filtration and washed with water and acetone. The crystals were dried by heating (45°C) to obtain 24.1 g of the compound 40 (yield: 77 % based on ethylenediamine).

### <Example wherein an alkali is used ― synthesis of compound 40 from compound 15>

100 ml of water and 19.2 g (0.48 mol) of sodium hydroxide were added to the reaction liquid obtained in Run No. 6 , from which the lanthanoid salt had not been filtered out. The obtained mixture was heated under reflux for 3 hours and then cooled. The pH of the reaction solution was adjusted to 2 with concentrated hydrochloric acid. After desalting by the electrodialysis, the reaction solution was concentrated under reduced pressure. After leaving the concentrate to stand in a refrigerator for one week, the crystals thus formed were taken by filtration and washed with water and acetone. The crystals were dried by heating (45 °C) to obtain 22.8 g of the compound 40 (yield: 73 % based on ethylenediamine).

The present invention can be considered to be suitable for the synthesis of the aminopolycarboxylic acids, since the nitrile group can be thus converted into the carboxyl group with an acid or alkali even without removing the lanthanoid salt.

### Example 2

The reaction-accelerating effect of an inorganic salt of lanthanum (La) obtained under the same reaction conditions as those of Example 1 was examined in comparison with that obtained without using the inorganic salt of lanthanum (La), that of La(OTf)₃ and that of lanthanum (La) oxide. The results are given in Table 2.

**Table 2**

| Lanthanum salt | Yield of compound 15 three hours after (%) |
|---|---|
| None | not higher than 6 % |
| LaCl₃ · 7H₂O | 90 |
| La(OTf)₃ | 72 |
| La₂O₃ | 89 |
| OTf: trifluoromethanesulfonate | |

It is understood from the results given in Table 2 that the reaction-accelerating effects of the inorganic salt and oxide of lanthanum (La) are far superior to that of La(OTf)₃.

### Example 3

### (Synthesis of various compounds)

7.0 g (0.12 mol) of ethylenediamine or 8.9 g (0.12 mol) of 1,3-propanediamine, a starting material shown in Table 3 (in an amount also shown in Table 3) and 0.43 g (1.2×10⁻³ mol) of LaCl₃ · 7H₂O were fed into a 50 ml short-neck flask and heated under reflux in an oil bath. Three hours after, the quantity of the intended product in the reaction solution was determined by high-performance liquid chromatography. The results are shown in Table 3.

**Table 3**

| Compound | Starting material | Amount | Yield after 3 hours (%)¹⁾ |
|---|---|---|---|
| Compound 14 | CH₃-CH=CH-CN | 0.35 mol | 93 |
| Compound 16 | CH₂=CH(CH₃)CN | 0.35 mol | 85 |
| Compound 17 | C₂H₅CH=CHCN | 0.35 mol | 89 |
| Compound 19 | CH₃CH=CHCN | 0.12 mol | 77 |
| Compound 20 | C₂H₅CH=CHCN | 0.12 mol | 74 |
| Compound 24 | Ph-CH=CHCN | 0.12 mol | 65 |
| Compound 27 | CH₂=CH(CH₃)CN | 0.12 mol | 71 |

| | | | |
|---|---|---|---|
| ¹⁾ Yield based on the amine Ph in Table 3 represents a phenyl group. | | | |

It is understood from the results shown in Table 3 that the accelerating effect can be obtained even by varying the α , *β*-unsaturated compound to be reacted.

### Example 4

7.0 g (0.12 mol) of ethylenediamine, 28.3 g (0.35 mol) of cis-2-pentenenitrile and LaCl₃ · 7H₂O in an amount shown in Table 4 were fed into a 50 ml short-neck flask and heated under reflux in an oil bath. Five hours after, the quantity of the compound 15 in the reaction solution was determined by high-performance liquid chromatography. The results are shown in Table 4.

**Table 4**

| Amount of lanthanum chloride¹⁾ | Yield of compound 15 after 5 hours (%)²⁾ |
|---|---|
| None | 6 % |
| 0.1 molar % | 73 % |
| 0.5 molar % | 89 % |
| 1.0 molar % | 95 % |
| 2.0 molar % | 92 % |
| 10 molar % | 81 % |

| | |
|---|---|
| ¹⁾ Molar % based on cis-2-pentenenitrile | |
| ²⁾ Yield based on ethylenediamine | |

It is understood that the accelerating effect of LaCl₃ · 7H₂O is satisfactory even though the yield varies a little depending on the amount thereof.

### Example 5

### (Synthesis of compound 29)

### 1) When a lanthanoid salt is used:

8.91 g (0.10 mol) of α-alanine and 11.61 g (0.10 mol) of maleic acid were fed into a three-necked flask. An aqueous solution of 12.0 g (0.3 mol) of sodium hydroxide in 25.5 g of water was added dropwise to the resultant mixture under thorough stirring. 18.55 g (0.05 mol) of lanthanum chloride heptahydrate was added to the reaction solution, and the resultant mixture was heated under reflux in an oil bath for 5 hours. The conversion as determined by NMR was not less than 95 %.

### 2) When no lanthanoid salt is used:

8.91 g (0.10 mol) of α-alanine and 11.61 g (0.10 mol) of maleic acid were fed into a three-necked flask. An aqueous solution of 12.0 g (0.3 mol) of sodium hydroxide in 25.5 g of water was added dropwise to the resultant mixture under thorough stirring. The resultant mixture was heated under reflux in an oil bath for 5 hours. The conversion as determined by NMR was 55 %.

It is understood from the results obtained as described above that the reaction-accelerating effect is obtained even when water having a high polarity is used as the solvent.

The method of the present invention is excellent in that the addition reaction of the amine with the α,β-unsaturated compound can be rapidly carried out to obtain a high yield of the adduct.

The compounds obtained by the method of the present invention are described in, for example, Japanese Patent Unexamined Published Application No. Hei 3-186841 (corresponding to U. S. Patent Nos. 5,238,791 and E. P. No. 430,000B), and usable in the fields of medical preparations, cosmetic preparations, soaps, detergents, cleaning compositions, material analysis, coating for metal materials, plating, catalysts, colloid chemistry, photography (such as developer and bleaching solution) and liquid crystals (such as chelating agents), as well as intermediates for them.

In the field of the photography, these compounds are usable, in the form of Fe complex salts, as bleaching agents, reducing agents, etc. in addition to the chelating agents.

## Claims

1. A method for producing an amine compound of the following general formula (I), which comprises the step of adding an amine compound (B) to an α,β-unsaturated compound (A) in the presence of an inorganic salt or oxide of a lanthanoid: 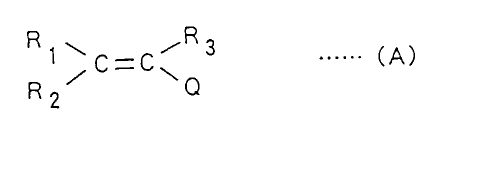 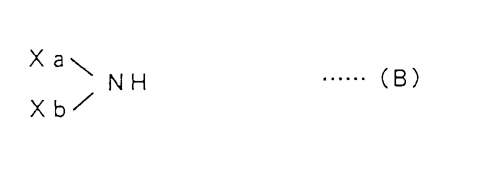 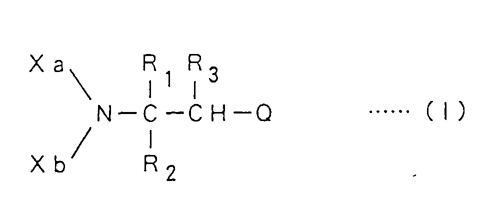 wherein R₁, R₂ and R₃ each represent a hydrogen atom or substituent, Q represents a nitrile group, carboxyl group, sulfonyl group, acyl group, carbamoyl group or nitro group, and Xa and Xb each represent a hydrogen atom or substituent.

2. The method of claim 1 wherein the substituent represented by each R₁, R₂, R₃, Xa and Xb is selected from the group consisting of aliphatic hydrocarbon groups, aryl groups, amino groups, alkoxy groups, aryloxy groups, acyl groups, alkoxycarbonyl groups, aryloxycarbonyl groups, acyloxy groups, acylamino groups, alkoxycarbonylamino groups, aryloxycarbonylamino groups, sulfonylamino groups, sulfamoyl groups, carbamoyl groups, alkylthio groups, arylthio groups, sulfonyl groups, sulfinyl groups, ureido groups, hydroxy group, mercapto group, halogen atoms, cyano group, sulfo group, carboxyl group, nitro group, hydroxamic acid group and heterocyclic groups.

3. The method of claim 1 wherein the lanthanoid is selected from the group consisting of lanthanum, cerium, praseodymium, neodymium, promethium, samarium, europium, gadolinium, terbium, dysprosium, holmium, erbium, thulium, ytterbium and lutetium.

4. The method of claim 1 wherein the inorganic salt is selected from the group consisting of chlorides, fluorides, iodides, bromides, nitrates, sulfates, carbonates and borides of a lanthanoid.

5. The method of claim 1 wherein the oxide is selected from the group consisting of lanthanum oxide, cerium oxide, neodymium oxide, samarium oxide, europium oxide, gadolinium oxide, terbium oxide, dysprosium oxide, holmium oxide, erbium oxide and ytterbium oxide.

6. The method of claim 1 wherein the salt or oxide of a lanthanoid is one recovered from a solution containing at least one of the amine compound (B) and the α,β-unsaturated compound (A).

7. The method of claim 1 wherein the α,β-unsaturated compound (A) is a compound (A₁₁) represented by the following formula: 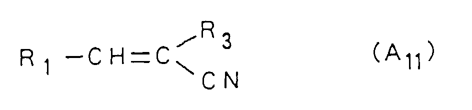 wherein R₁ and R₃ each represent a hydrogen atom, an aliphatic hydrocarbon group, aryl group, amino group, alkoxy group, aryloxy group, acyl group, alkoxycarbonyl group, aryloxycarbonyl group, acyloxy group, acylamino group, alkoxycarbonylamino group, aryloxycarbonylamino group, sulfonylamino group, sulfamoyl group, carbamoyl group, alkylthio group, arylthio group, sulfonyl group, sulfinyl group, ureido group, hydroxy group, mercapto group, halogen atoms, cyano group, sulfo group, carboxyl group, nitro group, hydroxamic acid group or heterocyclic group.

8. The method of claim 1 wherein the α,β-unsaturated compound (A) is a compound (A₁₁) represented by the following formula: 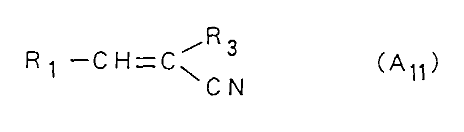 wherein R₁ and R₃ each represent a hydrogen atom, an alkyl having 1 to 12 carbon atoms, aryl having 6 to 20 carbon atoms, alkoxy having 1 to 8 carbon atoms, carboxyl, hydroxy, 5-membered or 6-membered aromatic heterocyclic group containing a nitrogen atom, nitrile or carbamoyl having 1 to 10 carbon atoms.

9. The method of claim 1 wherein the amine compound (B) is an amine (B₁ₐ) represented by the following formula: 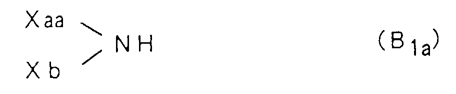 wherein Xaa represents an alkyl group, aryl group or heterocyclic group and Xb represents a hydrogen atom, an aliphatic hydrocarbon group, aryl group, amino group, alkoxy group, aryloxy group, acyl group, alkoxycarbonyl group, aryloxycarbonyl group, acyloxy group, acylamino group, alkoxycarbonylamino group, aryloxycarbonylamino group, sulfonylamino group, sulfamoyl group, carbamoyl group, alkylthio group, arylthio group, sulfonyl group, sulfinyl group, ureido group, hydroxy group, mercapto group, halogen atoms, cyano group, sulfo group, carboxyl group, nitro group, hydroxamic acid group or heterocyclic group.

10. The method of claim 1 wherein the amine compound (B) is an amine (B_{1b}) represented by the following formula: 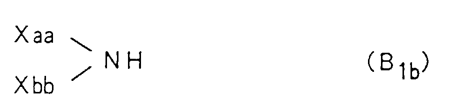 wherein Xaa represents an alkyl group, aryl group or heterocyclic group and Xbb represents a hydrogen atom or a group represented by the following formula Xaaa: 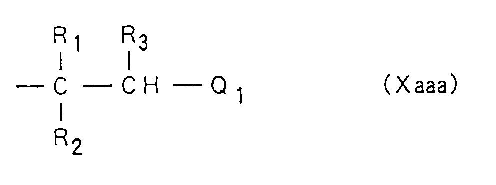 wherein R₁, R₂ and R₃ each represent an alkyl group, aryl group or heterocyclic group and Xb represents a hydrogen atom, an aliphatic hydrocarbon group, aryl group, amino group, alkoxy group, aryloxy group, acyl group, alkoxycarbonyl group, aryloxycarbonyl group, acyloxy group, acylamino group, alkoxycarbonylamino group, aryloxycarbonylamino group, sulfonylamino group, sulfamoyl group, carbamoyl group, alkylthio group, arylthio group, sulfonyl group, sulfinyl group, ureido group, hydroxy group, mercapto group, halogen atoms, cyano group, sulfo group, carboxyl group, nitro group, hydroxamic acid group or heterocyclic group, and Q represents a nitrile group, carboxyl group, sulfonyl group, acyl group, carbamoyl group or nitro group.

11. The method of claim 1 wherein the amine compound (B) is an amine (B_{1c}) represented by the following formula:
Xaa-NH₂ (B_{1c})
wherein Xaa is represents an alkyl group, aryl group or heterocyclic group.

12. The method of claim 1 wherein the amine compound (B) is an amine (B²) represented by the following formula: 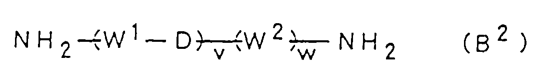 wherein W¹ and W² each represent an alkylene group, arylene group, aralkylene group or divalent nitrogen-containing hetero cyclic group, D represents a single bond, -O-, -S-, or -N(Rw)-, Rw being a hydrogen atom, aliphatic hydrocarbon group, aryl group or heterocyclic group, v represents an integer of 0 to 3, and w represents an integer of 1 to 3.

13. A method according to claim 1, wherein the amine (I) produced has the following formula (III), the amine compound (B) has the following formula (B_{1c}) and the α,β-unsaturated compound (A) has the following formula (A₁₁), and wherein the amine compound (B_{1c}) is added to the α,β-unsaturated compound (A₁₁) in the presence of an inorganic salt or oxide of a lanthanoid at a temperature of 10 to 200 °C: 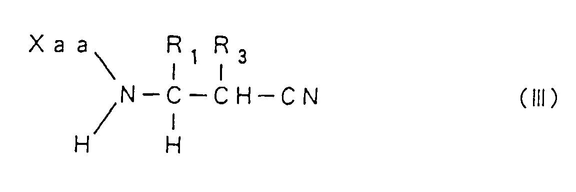 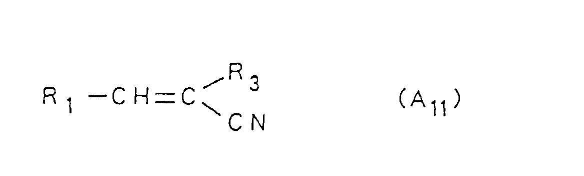
Xaa-NH₂ (B_{1c})
wherein R₁ and R₃ each represent a hydrogen atom, an alkyl having 1 to 12 carbon atoms, aryl having 6 to 20 carbon atoms, alkoxy having 1 to 8 carbon atoms, carboxyl, hydroxy, 5-membered or 6-membered aromatic heterocyclic ring containing a nitrogen atom, nitrile or carbamoyl having 1 to 10 carbon atoms, and Xaa represents an alkyl group having 1 to 20 carbon atoms.

14. A method according to claim 1, wherein the amine(I) produced is a diamine compound of the following general formula (II) and the amine compound (B) is a diamine of the following formula: 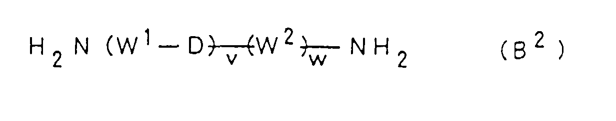 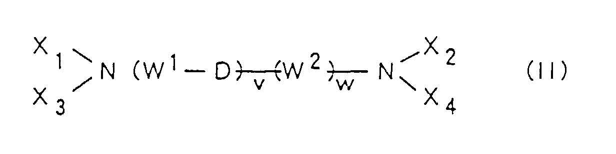 wherein W¹ and W² each represents an alkylene group, arylene group, aralkylene group or divalent nitrogen-containing heterocyclic group, D represents a single bond, -O-, -S- or -N(Rw)-, Rw being a hydrogen atom, aliphatic hydrocarbon group, aryl group or heterocyclic group, v represents an integer of 0 to 3, w represents an integer of 1 to 3, and X₁, X₂, X₃ and X₄ each represent a hydrogen atom or Xaaa, with the proviso that all of X₁, X₂, X₃ and X₄ cannot be hydrogen atom at the same time 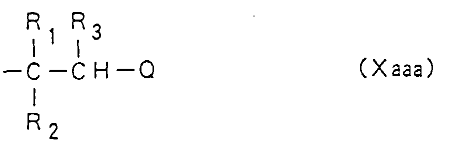 wherein R₁, R₂, R₃ and Q are as defined in claim 1

15. The method of claim 14 wherein the α, β -unsaturated compound (A) is a compound (A₁₁) represented by the following formula 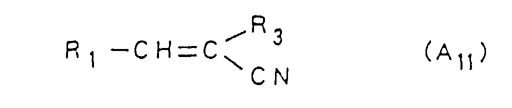 wherein R₁ and R₃ each represent a hydrogen atom, an alkyl having 1 to 12 carbon atoms, aryl having 6 to 20 carbon atoms, alkoxy having 1 to 8 carbon atoms, carboxyl, hydroxy, 5-membered or 6-membered aromatic heterocyclic group containing a nitrogen atom, nitrile or carbamoyl having 1 to 10 carbon atoms.

## Patentansprüche

1. Verfahren zur Herstellung einer Aminverbindung der folgenden allgemeinen Formel (I), welches den Schritt des Zusatzes einer Aminverbindung (B) zu einer α, β-ungesättigten Verbindung (A) in Gegenwart eines anorganischen Salzes oder Oxids eines Lanthanoids umfasst: 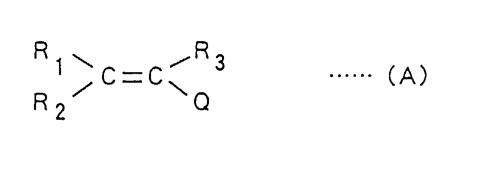 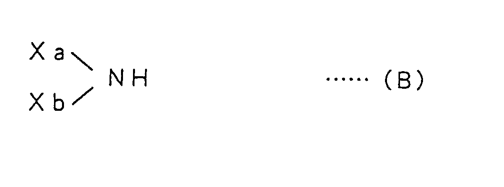 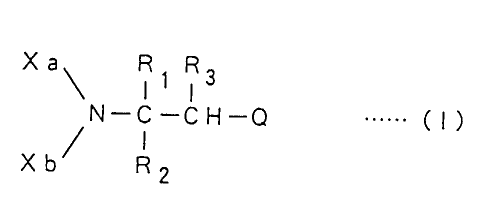 worin R₁, R₂ und R₃ jeweils für ein Wasserstoffatom oder einen Substituenten stehen, Q für eine Nitril-, Carboxyl-, Sulfonyl-, Acyl-, Carbamoyl- oder Nitrogruppe steht, und Xa und Xb jeweils für ein Wasserstoffatom oder einen Substituenten stehen.

2. Verfahren gemäß Anspruch 1, wobei der jeweils durch R₁, R₂, R₃, Xa und Xb wiedergegebene Substituent ausgewählt ist aus der Gruppe, bestehend aus aliphatischen Kohlenwasserstoffgruppen, Arylgruppen, Aminogruppen, Alkoxygruppen, Aryloxygruppen, Acylgruppen, Alkoxycarbonylgruppen, Aryloxycarbonylgruppen, Acyloxygruppen, Acylaminogruppen, Alkoxycarbonylaminogruppen, Aryloxycarbonylaminogruppen, Sulfonylaminogruppen, Sulfamoylgruppen, Carbamoylgruppen, Alkylthiogruppen, Arylthiogruppen, Sulfonylgruppen, Sulfinylgruppen, Ureidogruppen, der Hydroxygruppe, Mercaptogruppe, Halogenatomen, Cyano-, Sulfo-, Carboxyl-, Nitro-, Hydroxyamsäuregruppe und heterocyclischen Gruppen.

3. Verfahren gemäß Anspruch 1, wobei das Lanthanoid ausgewählt ist aus der Gruppe, bestehend aus Lanthan, Cer, Praseodym, Neodym, Promethium, Samarium, Europium, Gadolinium, Terbium, Dysprosium, Holmium, Erbium, Thulium, Ytterbium und Lutetium.

4. Verfahren gemäß Anspruch 1, wobei das anorganische Salz ausgewählt ist aus der Gruppe, bestehend aus Chloriden, Fluoriden, Iodiden, Bromiden, Nitraten, Sulfaten, Carbonaten und Boriden eines Lanthanoids.

5. Verfahren gemäß Anspruch 1, wobei das Oxid ausgewählt ist aus der Gruppe, bestehend aus Lanthan-, Cer-, Neodym-, Samarium-, Europium-, Gadolinium-, Terbium-, Dysprosium-, Holmium-, Erbium- und Ytterbiumoxid.

6. Verfahren gemäß Anspruch 1, wobei das Salz oder Oxid eines Lanthanoids eines ist, welches aus einer Lösung wiedergewonnen wird, die mindestens eine der Aminverbindung (B) und der α, β-ungesättigten Verbindung (A) enthält.

7. Verfahren gemäß Anspruch 1, wobei die α, β-ungesättigte Verbindung (A) eine Verbindung (A₁₁) mit der folgenden Formel ist: 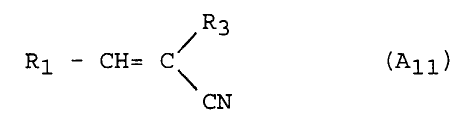 worin R₁ und R₃ jeweils stehen für ein Wasserstoffatom,eine aliphatische Kohlenwasserstoffgruppe, Arylgruppe, Aminogruppe, Alkoxygruppe, Aryloxygruppe, Acylgruppe, Alkoxycarbonylgruppe, Aryloxycarbonylgruppe, Acyloxygruppe, Acylaminogruppe, Alkoxycarbonylaminogruppe, Aryloxycarbonylaminogruppe, Sulfonylaminogruppe, Sulfamoylgruppe, Carbamoylgruppe, Alkylthiogruppe, Arylthiogruppe, Sulfonylgruppe, Sulfinylgruppe, Ureidogruppe, Hydroxygruppe, Mercaptogruppe, Halogenatome, Cyano-, Sulfo-, Carboxyl-, Nitro-, Hydroxamsäure- oder heterocyclische Gruppe.

8. Verfahren gemäß Anspruch 1, wobei die α, β-ungesättigte Verbindung (A) eine Verbindung (A₁₁) mit der folgenden Formel ist: 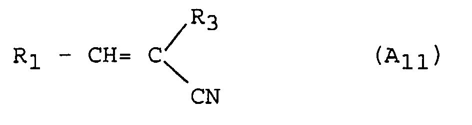 worin R₁ und R₃ jeweils stehen für ein Wasserstoffatom, ein Alkyl mit 1 bis 12 Kohlenstoffatomen, ein Aryl mit 6 bis 20 Kohlenstoffatomen, Alkoxy mit 1 bis 8 Kohlenstoffatomen, Carboxyl, Hydroxy, eine 5-gliedrige oder 6-gliedrige aromatische, ein Stickstoffatom enthaltende heterocyclische Gruppe, Nitril oder Carbamoyl mit 1 bis 10 Kohlenstoffatomen.

9. Verfahren gemäß Anspruch 1, wobei die Aminverbindung (B) eine Aminverbindung (B₁ₐ) mit der folgenden Formel ist: 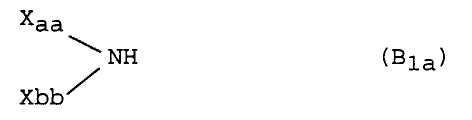 worin Xaa steht für eine Alkyl-, Aryl- oder heterocyclische Gruppe, und Xb steht für ein Wasserstoffatom, eine aliphatische Kohlenwasserstoffgruppe, Arylgruppe, Aminogruppe, Alkoxygruppe, Aryloxygruppe, Acylgruppe, Alkoxycarbonylgruppe, Aryloxycarbonylgruppe, Acyloxygruppe, Acylaminogruppe, Alkoxycarbonylaminogruppe, Aryloxycarbonylaminogruppe, Sulfonylaminogruppe, Sulfamoylgruppe, Carbamoylgruppe, Alkylthiogruppe, Arylthiogruppe, Sulfonylgruppe, Sulfinylgruppe, Ureidogruppe, Hydroxygruppe, Mercaptogruppe, Halogenatome, Cyano-, Sulfo-, Carboxyl-, Nitro-, Hydroxamsäure- oder heterocyclische Gruppe.

10. Verfahren gemäß Anspruch 1, wobei die Aminverbindung (B) eine Aminverbindung (B_{1b}) mit der folgenden Formel ist: 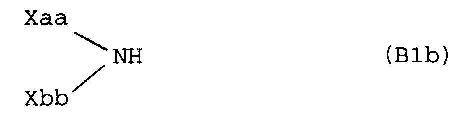 worin Xaa steht für eine Alkyl-, Aryl- oder heterocyclische Gruppe und Xbb steht für ein Wasserstoffatom oder eine Gruppe mit der folgenden Formel Xaaa: 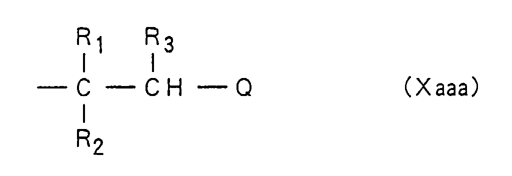 worin R₁, R₂ und R₃ jeweils stehen für eine Alkyl-, Aryl- oder heterocyclische Gruppe und Xb steht für ein Wasserstoffatom, eine aliphatische Kohlenwasserstoffgruppe, Arylgruppe, Aminogruppe, Alkoxygruppe, Aryloxygruppe, Acylgruppe, Alkoxycarbonylgruppe, Aryloxycarbonylgruppe, Acyloxygruppe, Acylaminogruppe, Alkoxycarbonylaminogruppe, Aryloxycarbonylaminogruppe, Sulfonylaminogruppe, Sulfamoylgruppe, Carbamoylgruppe, Alkylthiogruppe, Arylthiogruppe, Sulfonylgruppe, Sulfinylgruppe, Ureidogruppe, Hydroxygruppe, Mercaptogruppe, Halogenatome, Cyano-, Sulfo-, Carboxyl-, Nitro-, Hydroxamsäure- oder heterocyclische Gruppe, und Q steht für eine Nitril-, Carboxyl-, Sulfonyl-, Acyl-, Carbamoyl- oder Nitrogruppe.

11. Verfahren gemäß Anspruch 1, wobei die Aminverbindung (B) ein Amin (B_{1c}) mit der folgenden Formel ist:
Xaa - NH₂ (B_{1c})
worin Xaa für eine Alkyl-, Aryl- oder heterocyclische Gruppe steht.

12. Verfahren gemäß Anspruch 1, wobei die Aminverbindung (B) ein Amin (B²) mit der folgenden Formel ist: 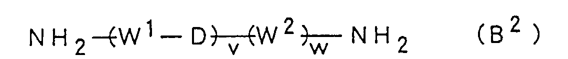 worin W¹ und W² jeweils stehen für eine Alkylen-, Arylen-, Aralkylen- oder divalente, stickstoffhaltige heterocyclische Gruppe, D für eine Einfachbindung -O-, -S- oder -N(Rw)- steht, wobei Rw ein Wasserstoffatom, eine aliphatische Kohlenwasserstoffgruppe, Arylgruppe oder heterocyclische Gruppe ist, v steht für eine ganze Zahl von 0 bis 3 und w steht für eine ganze Zahl von 1 bis 3.

13. Verfahren gemäß Anspruch 1, wobei das hergestellte Amin (I) die folgende Formel (III), die Aminverbindung (B) die folgende Formel (B_{1c}) und die α, β-ungesättigte Verbindung (A) die folgende Formel (A₁₁) hat, und worin die Aminverbindung (B_{1c}) zur α, β-ungesättigten Verbindung (A₁₁) in Gegenwart eines anorganischen Salzes oder Oxids eines Lanthanoids bei einer Temperatur von 10 bis 200°C zugesetzt wird: 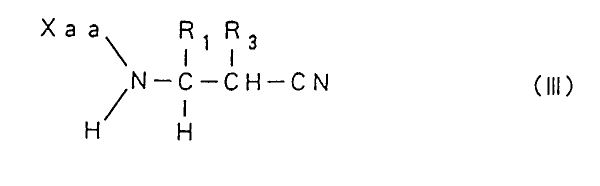 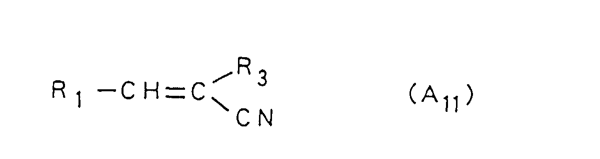
Xaa-NH₂ (B_{1c})
worin R₁ und R₃ jeweils stehen für ein Wasserstoffatom, ein Alkyl mit 1 bis 12 Kohlenstoffatomen, Aryl mit 6 bis 20 Kohlenstoffatomen, Alkoxy mit 1 bis 8 Kohlenstoffatomen, Carboxyl, Hydroxy, einen 5-gliedrigen oder 6-gliedrigen aromatischen, ein Stickstoffatom enthaltenden heterocyclischen Ring, Nitril oder Carbamoyl mit 1 bis 10 Kohlenstoffatomen, und Xaa steht für eine Alkylgruppe mit 1 bis 20 Kohlenstoffatomen.

14. Verfahren gemäß Anspruch 1, wobei das hergestellte Amin (I) eine Diaminverbindung der folgenden allgemeinen Formel (II) ist, und die Aminverbindung (B) ein Diamin mit der folgenden Formel ist: 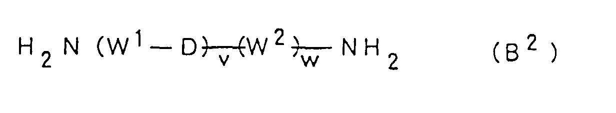 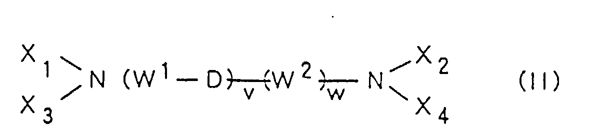 worin W¹ und W² jeweils stehen für eine Alkylen-, Arylen-, Aralkylen- oder eine divalente, stickstoffhaltige heterocyclische Gruppe, D steht für eine Einfachbindung, -O-, -S- oder -N(Rw)-, wobei Rw ein Wasserstoffatom, aliphatische Kohlenwasserstoff-, Aryl- oder heterocyclische Gruppe ist, v steht für eine ganze Zahl von 0 bis 3, w steht für eine ganze Zahl von 1 bis 3, und X₁, X₂, X₃ und X₄ stehen jeweils für ein Wasserstoffatom oder Xaaa, mit der Maßgabe, dass X₁, X₂, X₃ und X₄ nicht alle gleichzeitig ein Wasserstoffatom sein können 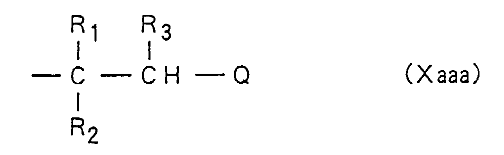 worin R₁, R₂, R₃ und Q wie in Anspruch 1 definiert sind.

15. Verfahren gemäß Anspruch 14, wobei die α, β-ungesättigte Verbindung (A) eine Verbindung (A₁₁) der folgenden Formel ist: 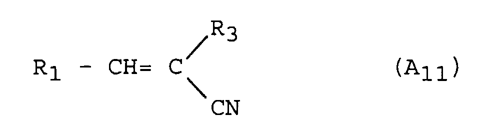 worin R₁ und R₃ jeweils stehen für ein Wasserstoffatom, ein Alkyl mit 1 bis 12 Kohlenstoffatomen, Aryl mit 6 bis 20 Kohlenstoffatomen, Alkoxy mit 1 bis 8 Kohlenstoffatomen, Carboxyl, Hydroxy, eine 5-gliedrige oder 6-gliedrige, ein Stickstoffatom enthaltende, aromatische, heterocyclische Gruppe, Nitril oder Carbamoyl mit 1 bis 10 Kohlenstoffatomen.

## Revendications

1. Procédé de production d'un composé de type amine répondant à la formule générale (I) suivante, qui comprend l'étape consistant à ajouter un composé de type amine (B) à un composé insaturé en α et β (A) en présence d'un sel ou oxyde inorganique d'un lanthanide : 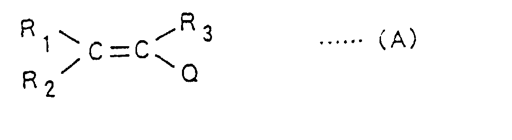 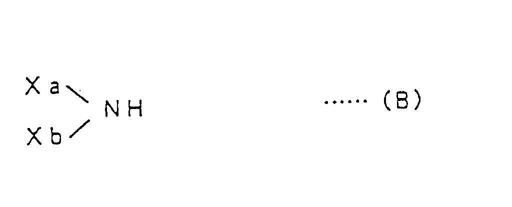 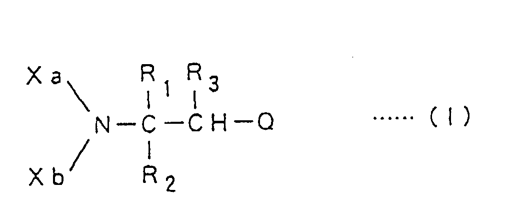 où R₁, R₂ et R₃ représentent chacun un atome d'hydrogène ou un substituant, Q représente un groupe nitrile, un groupe carboxyle, un groupe sulfonyle, un groupe acyle, un groupe carbamoyle ou un groupe nitro, et Xa et Xb représentent chacun un atome d'hydrogène ou un substituant.

2. Procédé selon la revendication 1, dans lequel le substituant représenté par chaque symbole R₁, R₂, R₃, Xa et Xb est choisi dans le groupe constitué par des groupes hydrocarbonés aliphatiques, des groupes aryle, des groupes amino, des groupes alcoxy, des groupes aryloxy, des groupes acyle, des groupes alcoxycarbonyle, des groupes aryloxycarbonyle, des groupes acyloxy, des groupes acylamino, des groupes alcoxycarbonylamino, des groupes aryloxycarbonylamino, des groupes sulfonylamino, des groupes sulfamoyle, des groupes carbamoyle, des groupes alkylthio, des groupes arylthio, des groupes sulfonyle, des groupes sulfinyle, des groupes uréido, le groupe hydroxy, le groupe mercapto, des atomes d'halogène, le groupe cyano, le groupe sulfo, le groupe carboxyle, le groupe nitro, le groupe acide hydroxamique et des groupes hétérocycliques.

3. Procédé selon la revendication 1, dans lequel le lanthanide est choisi dans le groupe constitué par le lanthane, le cérium, le praséodyme, le néodyme, le prométhium, le samarium, l'europium, le gadolinium, le terbium, le dysprosium, l'holmium, l'erbium, le thulium, l'ytterbium et le lutétium.

4. Procédé selon la revendication 1, dans lequel le sel inorganique est choisi dans le groupe constitué par les chlorures, les fluorures, les iodures, les bromures, les nitrates, les sulfates, les carbonates et les borures de lanthanide.

5. Procédé selon la revendication 1, dans lequel l'oxyde est choisi dans le groupe constitué par l'oxyde de lanthane, l'oxyde de cérium, l'oxyde de néodyme, l'oxyde de samarium, l'oxyde d'europium, l'oxyde de gadolinium, l'oxyde de terbium, l'oxyde de dysprosium, l'oxyde d'holmium, l'oxyde d'erbium et l'oxyde d'ytterbium.

6. Procédé selon la revendication 1, dans lequel le sel ou oxyde d'un lanthanide est récupéré à partir d'une solution contenant au moins un des composés parmi le composé de type anime (B) et le composé insaturé en α et β (A).

7. Procédé selon la revendication 1, dans lequel le composé insaturé en α et β (A) est un composé (A₁₁) représenté par la formule suivante : 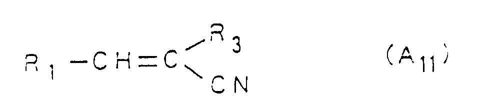 dans laquelle R₁ et R₃ représentent chacun un atome d'hydrogène, un groupe hydrocarboné aliphatique, un groupe aryle, un groupe amino, un groupe alcoxy, un groupe aryloxy, un groupe acyle, un groupe alcoxycarbonyle, un groupe aryloxycarbonyle, un groupe acyloxy, un groupe acylamino, un groupe alcoxycarbonylamino, un groupe aryloxycarbonylamino, un groupe sulfonylamino, un groupe sulfamoyle, un groupe carbamoyle, un groupe alkylthio, un groupe arylthio, un groupe sulfonyle, un groupe sulfinyle, un groupe uréido, un groupe hydroxy, un groupe mercapto, des atomes d'halogène, un groupe cyano, un groupe sulfo, un groupe carboxyle, un groupe nitro, un groupe acide hydroxamique ou un groupe hétérocyclique.

8. Procédé selon la revendication 1, dans lequel le composé insaturé en α et β (A) est un composé (A₁₁) représenté par la formule suivante : 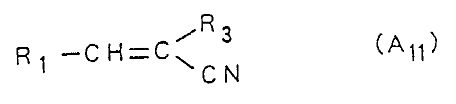 dans laquelle R₁ et R₃ représentent chacun un atome d'hydrogène, un groupe alkyle comportant 1 à 12 atomes de carbone, un groupe aryle comportant 6 à 20 atomes de carbone, un groupe alcoxy comportant 1 à 8 atomes de carbone, un groupe carboxyle, un groupe hydroxy, un groupe hétérocyclique aromatique à 5 ou 6 chaînons contenant un atome d'azote, un groupe nitrile ou un groupe carbamoyle comportant 1 à 10 atomes de carbone.

9. Procédé selon la revendication 1, dans lequel le composé de type amine (B) est une amine (B₁ₐ) représentée par la formule suivante : 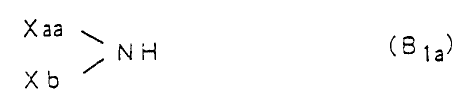 dans laquelle Xaa représente un groupe alkyle, un groupe aryle ou un groupe hétérocyclique et Xb représente un atome d'hydrogène, un groupe hydrocarboné aliphatique, un groupe aryle, un groupe amino, un groupe alcoxy, un groupe aryloxy, un groupe acyle, un groupe alcoxycarbonyle, un groupe aryloxycarbonyle, un groupe acyloxy, un groupe acylamino, un groupe alcoxycarbonylamino, un groupe aryloxycarbonylamino, un groupe sulfonylamino, un groupe sulfamoyle, un groupe carbamoyle, un groupe alkylthio, un groupe arylthio, un groupe sulfonyle, un groupe sulfinyle, un groupe uréido, un groupe hydroxy, un groupe mercapto, des atomes d'halogène, un groupe cyano, un groupe sulfo, un groupe carboxyle, un groupe nitro, un groupe acide hydroxamique ou un groupe hétérocyclique.

10. Procédé selon la revendication 1, dans lequel le composé de type amine (B) est une amine (B_{1b}) représentée par la formule suivante : 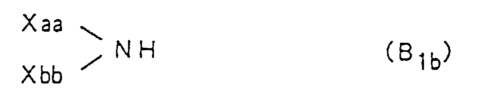 dans laquelle Xaa représente un groupe alkyle, un groupe aryle ou un groupe hétérocyclique et Xbb représente un atome d'hydrogène ou un groupe représenté par la formule Xaaa suivante : 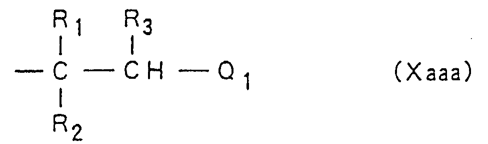 dans laquelle R₁, R₂ et R₃ représentent chacun un groupe alkyle, un groupe aryle ou un groupe hétérocyclique et Xb représente un atome d'hydrogène, un groupe hydrocarboné aliphatique, un groupe aryle, un groupe amino, un groupe alcoxy, un groupe aryloxy, un groupe acyle, un groupe alcoxyarbonyle, un groupe aryloxycarbonyle, un groupe acyloxy, un groupe acylamino, un groupe alcoxycarbonylamino, un groupe aryloxycarbonylamino, un groupe sulfonylamino, un groupe sulfamoyle, un groupe carbamoyle, un groupe alkylthio, un groupe arylthio, un groupe sulfonyle, un groupe sulfinyle, un groupe uréido, un groupe hydroxy, un groupe mercapto, des atomes d'halogène, un groupe cyano, un groupe sulfo, un groupe carboxyle, un groupe nitro, un groupe acide hydroxamique ou un groupe hétérocyclique, et Q représente un groupe nitrile, un groupe carboxyle, un groupe sulfonyle, un groupe acyle, un groupe carbamoyle ou un groupe nitro.

11. Procédé selon la revendication 1, dans lequel le composé de type amine (B) est une amine (B_{1c}) représentée par la formule suivante :
Xaa-NH₂ (B_{1c})
dans laquelle Xaa représente un groupe alkyle, un groupe aryle ou un groupe hétérocyclique.

12. Procédé selon la revendication 1, dans lequel le composé de type amine (B) est une amine (B²) représentée par la formule suivante : 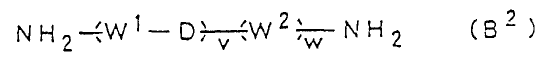 dans laquelle W¹ et W² représentent chacun un groupe alkylène, un groupe arylène, un groupe aralkylène ou un groupe hétérocyclique divalent contenant de l'azote, D représente une simple liaison, -O-, -S- ou -N(Rw)-, Rw représentant un atome d'hydrogène, un groupe hydrocarboné aliphatique, un groupe aryle ou un groupe hétérocyclique, v représente un nombre entier de 0 à 3, et w représente un nombre entier de 1 à 3.

13. Procédé selon la revendication 1, dans lequel l'amine (I) produite répond à la formule (III) suivante, le composé de type amine (B) répond à la formule (B_{1c}) suivante et le composé insaturé en α et β (A) répond à la formule (A₁₁) suivante, et dans lequel on ajoute le composé de type amine (B_{1c}) au composé insaturé en α et β (A₁₁) en présence d'un sel ou oxyde inorganique d'un lanthanide à une température de 10 à 200°C : 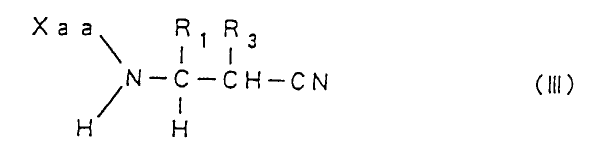 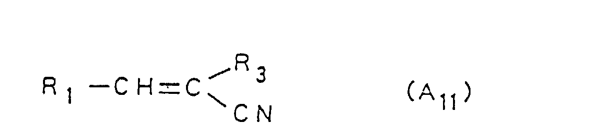
Xaa-NH₂ (B_{1c})
où R₁ et R₃ représentent chacun un atome d'hydrogène, un groupe alkyle comportant 1 à 12 atomes de carbone, un groupe aryle comportant 6 à 20 atomes de carbone, un groupe alcoxy comportant 1 à 8 atomes de carbone, un groupe carboxyle, un groupe hydroxy, un groupe hétérocyclique aromatique à 5 ou 6 chaînons contenant un atome d'azote, un groupe nitrile ou un groupe carbamoyle comportant 1 à 10 atomes de carbone, et Xaa représente un groupe alkyle comportant 1 à 20 atomes de carbone.

14. Procédé selon la revendication 1, dans lequel l'amine (I) produite est un composé de type diamine répondant à la formule générale (II) suivante et le composé de type amine (B) est une diamine répondant à la formule suivante : 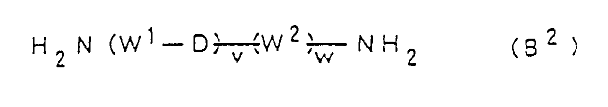 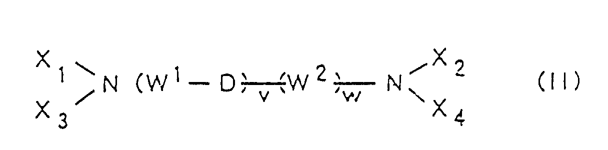 où W¹ et W² représentent chacun un groupe alkylène, un groupe arylène, un groupe aralkylène ou un groupe hétérocyclique divalent contenant de l'azote, D représente une simple liaison, -0-, -S- ou -N(Rw)-, Rw représentant un atome d'hydrogène, un groupe hydrocarboné aliphatique, un groupe aryle ou un groupe hétérocyclique, v représente un nombre entier de 0 à 3, w représente un nombre entier de 1 à 3, et X₁, X₂, X₃ et X₄ représentent chacun un atome d'hydrogène ou Xaaa, sous réserve que tous les symboles X₁, X₂, X₃ et X₄ ne puissent pas représenter en même temps un atome d'hydrogène 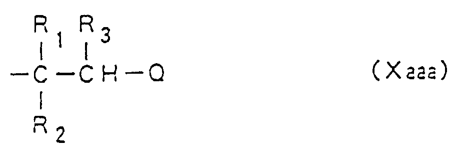 où R₁, R₂, R₃ et Q sont tels que définis dans la revendication 1.

15. Procédé selon la revendication 14, dans lequel le composé insaturé en α et β (A) est un composé (A₁₁) représenté par la formule suivante : 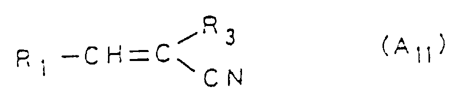 dans laquelle R₁ et R₃ représentent chacun un atome d'hydrogène, un groupe alkyle comportant 1 à 12 atomes de carbone, un groupe aryle comportant 6 à 20 atomes de carbone, un groupe alcoxy comportant 1 à 8 atomes de carbone, un groupe carboxyle, un groupe hydroxy, un groupe hétérocyclique aromatique à 5 ou 6 chaînons contenant un atome d'azote, un groupe nitrile ou un groupe carbamoyle comportant 1 à 10 atomes de carbone.
